(19) Europäisches Patentamt
European Patent Office
Office européen des brevets

(11) EP 0 303 279 B1

# (12) EUROPEAN PATENT SPECIFICATION

(45) Date of publication and mention of the grant of the patent:
**25.09.1996 Bulletin 1996/39**

(51) Int Cl.[6]: **C07D 211/58**, C08K 5/34

(21) Application number: **88113132.0**

(22) Date of filing: **12.08.1988**

## (54) Reactive hindered amine light stabilizers

Reaktive Amin-Leuchtstabilisatoren

Stabilisants aminés réactifs pour la lumière

(84) Designated Contracting States:
**BE CH DE ES FR GB IT LI NL SE**

(30) Priority: **12.08.1987 US 84602**

(43) Date of publication of application:
**15.02.1989 Bulletin 1989/07**

(73) Proprietor: **ELF ATOCHEM NORTH AMERICA, INC.**
**Philadelphia Pennsylvania 19102-3222 (US)**

(72) Inventors:
- **MacLeay, Ronald Edward**
**Williamsville New York 14221 (US)**
- **Kazmierczak, Robert Thaddeus**
**Williamsville New York 14221 (US)**

(74) Representative: **Abitz, Walter, Dr.-Ing. et al**
**Patentanwälte Abitz & Partner**
**Postfach 86 01 09**
**81628 München (DE)**

(56) References cited:
**EP-A- 0 002 260**
**US-A- 4 191 683**
**US-A- 4 223 147**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 303 279 B1

## Description

Background of the Invention

This invention relates to N-(2,2,6,6-tetraalkyl-4-piperidinyl)amide-hydrazides of dicarboxylic acids which contain a hindered amine light stabilizing group (photooxidative stabilizer) and a reactive hydrazide group (heat stabilizer).

Polymers such as polyolefins (e.g., polyethylene, polypropylene, etc.) styrenics (e.g., polystyrene, rubber modified polystyrene, ABS, MBS, etc.), polyvinyl chloride, polycarbonates, polyesters, polyphenylene ethers, and polyamides, for example, are subject to degradation and discoloration upon exposure to heat and/or light with consequent deterioration of their mechanical properties.

Various stabilizers have been proposed to inhibit such deterioration. Hindered piperidine compounds have found extensive use in the photostabilization of polyolefins. Hydrazides have been used to prevent deterioration of polyolefins by heat, oxidation, or heavy metal contamination. Derivatives of hydrazides are also commercially available for use as polymer stabilizers. (See Encyclopedia of Polymer Science and Engineering, 2nd Ed. Vol. 2, pp 83-84).

Four examples were found in the literature where the hindered amine moiety and the hydrazide moiety (-R-C(=O)-NH-$NH_2$, where R is not O, N, or S) are present in the same molecule.

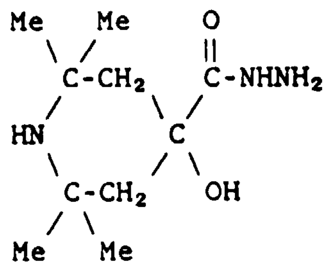

CA Registry Number 66651-22-7
U.S. Patents 4,153,596 and 4,223,147

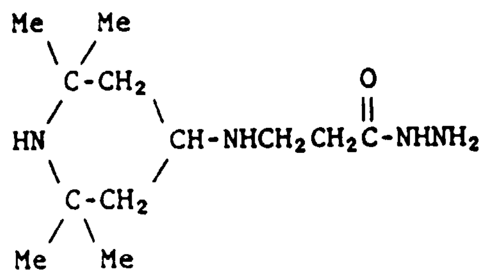

CA Registry Number 66651-23-8
U.S. Patents 4,153,596 and 4,223,147

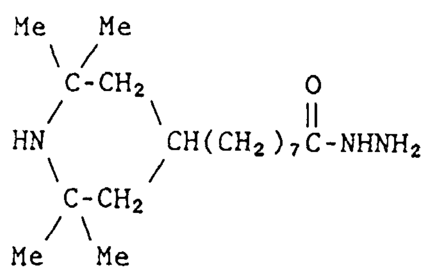

CA Registry Number 72436-11-4
JP 79/103461; CA92:59703j
JP 79/95649; CA92:42845j

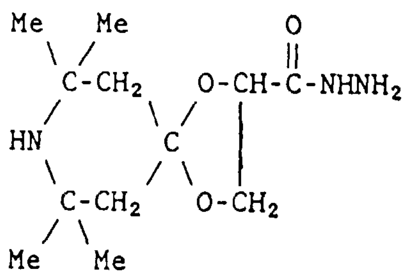

CA Registry Number 77246-76-5
U.S. Patent 4,336,183; CA97:217369g
E. P. Appl. 22997; CA94:176176s

None of these prior art hydrazides fall under general structure I of the present invention.

Sterically hindered 4-aminopiperidine derivatives which can be used as light-stabilizers for organic polymers are known from U.S. Patent 4,191,683.

SUMMARY OF THE INVENTION

This invention is directed to reactive N-(2,2,6,6-tetraalkyl-4-piperidinyl)-amide hydrazides having the following formula:

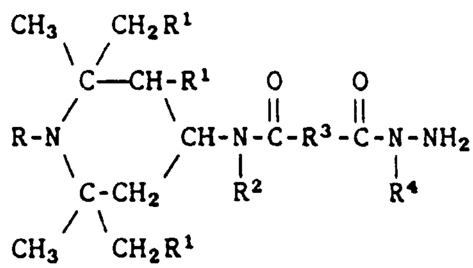

I

where

R is selected from hydrogen, oxyl, hydroxyl, alkyl of 1 to 20 carbons, alkenyl or alkynyl of 3 to 8 carbons, aralkyl of 7 to 12 carbons, aliphatic acyl of 1 to 10 carbons, aromatic acyl of 7 to 13 carbons, alkoxycarbonyl of 2 to 9 carbons, aryloxycarbonyl of 7 to 15 carbons, alkyl, aryl, cycloalkyl or aralkyl substituted carbamoyl of 2 to 13 carbons, 2-cyanoethyl, hydroxyalkyl of 1 to 6 carbons, epoxyalkyl of 3 to 10 carbons or a polyalkylene oxide group of 4 to 30 carbons.

$R^1$ is selected from hydrogen or lower alkyl of 1 to 4 carbons.

$R^2$ is selected from hydrogen, alkyl of 1 to 10 carbons, cycloalkyl of 5 to 12 carbons, aralkyl of 7 to 12 carbons, aryl of 6 to 12 carbons, 2-cyanoethyl or a radical of the formula

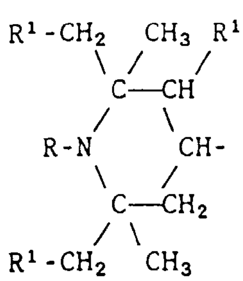

,

$R^3$ is selected from a direct bond, an alkylene or branched alkylene diradical of 1 to 14 carbons, an alkenylene diradical of 2 to 10 carbons, an oxydialkylene or thiodialkylene diradical of 4 to 10 carbons or an o-, m- or p-phenylene or substituted phenylene diradical where the substituents may be $C_1$-$C_4$ alkyl, $C_1$-$C_4$ alkoxy, hydroxy,

bromine, chlorine, mercapto or $C_1$-$C_4$ alkylmercapto.

$R^4$ is selected from hydrogen, a primary or secondary alkyl of 1 to 8 carbons, an aralkyl of 7 to 12 carbons or a cycloalkyl of 5 to 12 carbons.

Especially, R is H, $C_{1-4}$ alkyl, $C_3$-alkenyl, benzyl, 2-cyanoethyl or acetyl, $R^1$ is H or $CH_3$, $R^2$ is H, $C_{1-4}$ alkyl or 2,2,6,6-tetramethyl-4-piperidinyl, $R^3$ is a direct bond, $C_{1-8}$ alkylene or o-, m- or p-phenylene and $R^4$ is H, primary or secondary $C_{1-4}$ alkyl, benzyl or cyclohexyl.

Preferably, R is hydrogen, acetyl or methyl, $R^1$ is hydrogen, $R^2$ is hydrogen or a 2,2,6,6-tetramethyl-4-piperidinyl radical, $R^4$ is H or $CH_3$, and $R^3$ is a direct bond or a 1,2-ethylene diradical. Most preferably, $R^3$ is a direct bond or $C_1$-$C_8$ alkylene, especially $-CH_2CH_2-$ and R, $R^1$, $R^2$ and $R^4$ are hydrogen.

This invention also comprehends the preparation of the compound of Formula I.

Method A

The N-(2,2,6,6-tetraalkyl-4-piperidinyl)amide-hydrazides of Formula I are prepared by reacting the 4-amino-2,2,6,6-tetraalkylpiperidines of Formula II with half ester-half acid chloride of Formula III to form the intermediate half ester-half amides of Formula IV. The intermediate half ester-half amides are then reacted with a primary or secondary alkyl hydrazine, hydrazine or hydrazine hydrate to form the compounds I.

Method B

The N-(2,2,6,6-tetraalkyl-4-piperidinyl)amide-hydrazides of Formula VIII may also be prepared by reacting the amino compounds of Formula II with essentially equivalent amounts or an excess of a dicarboxylic acid diester of Formula VI to form the intermediate half ester-half amide of Formula VII. The intermediate VII is then reacted with a primary or secondary alkylhydrazine, hydrazine or hydrazine hydrate to form the compounds I with the limitation $R^3$ may not be an alkenylene or ethylene diradical.

Detailed Description Of The Invention

The compounds I are reactive additives that can be attached to coreactive polymers to form polymer bound additives containing photooxidative and thermal oxidative stabilizing groups. For example, the reactive N-(2,2,6,6-tetraalkyl-4-piperidinyl)amide-hydrazides can be reacted with anhydride copolymers such as styrene-maleic (SMA) copolymers, octadecene-maleic anhydride copolymer and ethylene-maleic anhydride copolymer to name just a few. Once reacted with the coreactive polymers the stabilizer groups become attached to the polymers and will not be lost via volatilization, migration or extraction.

Examples of the reactive hindered amine light stabilizers of this invention include the following non limiting list of hydrazides:

1. N-(1,2,2,6,6-pentamethyl-4-piperidinyl)-N′-aminooxamide,
2. N-(1-benzoyl-2,2,6,6-tetramethyl-4-piperidinyl)-N′-aminooxamide,
3. N-(1-allyl-2,2,6,6-tetramethyl-4-piperidinyl)-N′-aminooxamide,
4. N-(1-benzyl-2,2,6,6-tetramethyl-4-piperidinyl)-N′-aminooxamide,
5. N-(1-ethoxycarbonyl-2,2,6,6-tetramethyl-4-piperidinyl)-N′-aminooxamide,
6. N-(1-dodecyl-2,6-diethyl-2,3,6-trimethyl-4-piperidinyl)-N′-aminooxamide,
7. N-(2,2,6,6-tetramethyl-4-piperidinyl)-N-butyl-N′-aminooxamide,
8. N-(2,2,6,6-tetramethyl-4-piperidinyl)-N-phenyl-N′-aminosuccinamide,
9. N-(2,2,6,6-tetramethyl-4-piperidinyl)-N-methyl-N′-methyl-N′-aminomalonamide,
10. N-(2,2,6,6-tetramethyl-4-piperidinyl)-N′-aminoterephthalamide,
11. N-(2,2,6,6-tetramethyl-4-piperidinyl)-N′-butyl-N′-aminosebacamide,
12. N-(2,2,6,6-tetramethyl-4-piperidinyl)-N′-aminododecandiamide,
13. N-(2,2,6,6-tetramethyl-4-piperidinyl)-N′-aminosuberic acid diamide,
14. N-(2,2,6,6-tetramethyl-4-piperidinyl)-N′-aminoglutaric acid diamide,
15. N-(2,2,6,6-tetramethyl-4-piperidinyl)-N′-amino-2-methylsuccinamide,
16 N-(2,2,6,6-tetramethyl-4-piperidinyl)-N′-amino-2,3-dimethylsuccinamide,
17. N-(2,2,6,6-tetramethyl-4-piperidinyl)-N′-aminopimelic acid diamide,
18. N-(2,2,6,6-tetramethyl-4-piperidinyl)-N′-aminoundecandiamide,
19. N-(1-beta-hydroxyethyl-2,2,6,6-tetramethyl-4-piperidinyl)-N′-aminoadipic acid diamide,
20. N(1-beta-cyanoethyl-2,2,6,6-tetramethyl-4-piperidinyl)-N′-aminosuccinamide,

21. N-(1-phenoxycarbonyl-2,2,6,6-tetramethyl-4-piperidinyl)-N′-aminooxamide,
22. N-(2,6-diethyl-2,3,6-trimethyl-4-piperidinyl)-N′-aminooxamide, and
23. N,N-bis-(2,2,6,6-tetramethyl-4-piperidinyl)-N′-aminooxamide.

Compounds I are prepared by Methods A and B

The reaction sequence of Method A is illustrated by the following equations.

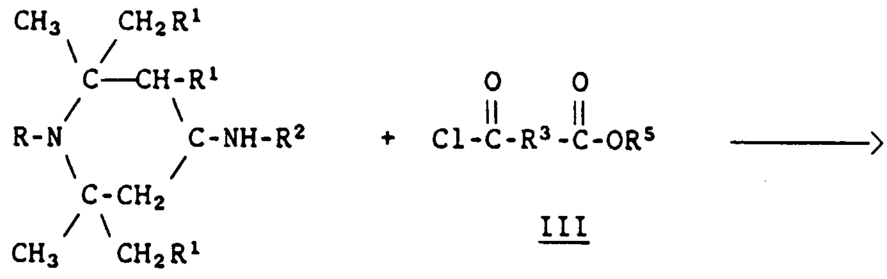

III

II

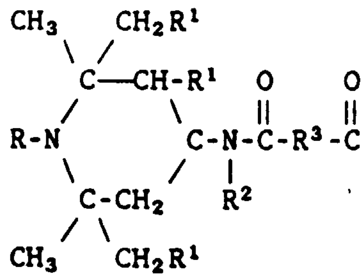

IV

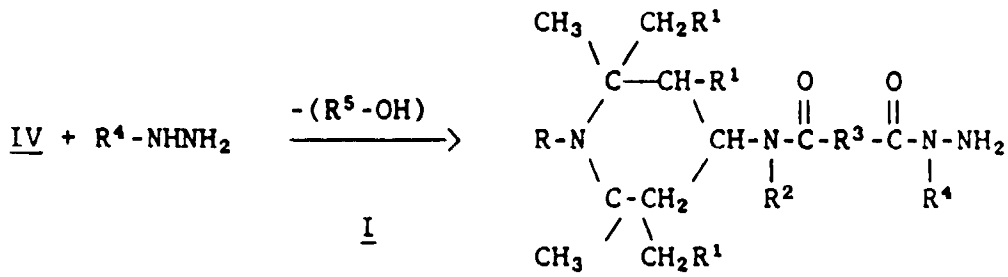


I

where R, $R^1$, $R^2$, $R^3$, and $R^4$ are as previously defined and $R^5$ is lower alkyl of 1 to 6 carbons or phenyl and preferably is methyl or ethyl.

Since HCl is liberated in the first step it is preferable to run the reaction in the presence of a hydrogen chloride acceptor such as a tertiary amine, an inorganic base or an excess of the amine II. Preferably, a 100% excess of the amine II is used as the hydrogen chloride acceptor. The amine hydrochloride formed can be separated from the reaction mixture by filtration or may be removed by washing the reaction mixture with water. (The amine can be regenerated by neutralization with a stronger base.) The reaction can be run in a variety of non-reactive solvents such as hydrocarbons, chlorinated hydrocarbons or ethers. Preferably, the reaction is run in the lower boiling solvents so the product can be isolated by stripping off the solvent. The reaction is exothermic so gentle cooling is advisable to control the temperature. The intermediate (IV) is isolated and dissolved in a polar solvent and converted to the desired hydrazide by stirring with an equivalent amount or slight excess of a primary or secondary-alkylhydrazine, hydrazine or hydrazine hydrate. Depending upon $R^3$, the reaction may go at room temperature or may require refluxing. Preferably, the hydrazinolysis reaction is carried out in methanol or ethanol at 10-30°C but other solvents such as isopropanol or ethylene glycol are also acceptable. In most cases the I compounds can be purified by recrystallization from the lower alcohols.

Examples of suitable mono ester acid chloride include: the mono esters (preferably the mono methyl or ethyl esters) of phthaloyl, isophthaloyl, terephthaloyl, adipoyl, azelayoyl, succinyl, oxalyl, malonyl, fumaroyl, sebacoyl and suberoyl

chlorides.

Examples of suitable amino-substituted hindered amine light stabilizers (II) include:

4-amino-2,2,6,6-tetramethylpiperidine,
4-amino-1,2,2,6,6-pentamethylpiperidine,
4-amino- 2,6-diethyl-2,3,6-trimethylpiperidine,
4-amino-2,6-diethyl- 1,2,3,6-tetramethylpiperidine,
4-amino-1-(2-cyanoethyl)-2,2,6,6-tetramethylpiperidine,
4-[N-(n-butyl)amino]-2,2,6,6-tetramethylpiperidine,
4-[N-(ethyl)amino]-1-ethoxycarbonyl-2,2,6,6-tetramethylpiperidine,
4-[N-(hexyl)amino]-1-benzyl-2,2,6,6-tetramethylpiperidine,
4-amino-1-allyl-2,2,6,6-tetramethylpiperidine,
4-amino-1-benzoyl-2,2,6,6-tetramethylpiperidine,
4-[N-(methyl)amino]-1-dodecyl-2,2,6,6-tetramethylpiperidine,
4-[N-(phenyl)amino]-1,2,2,6,6-pentamethylpiperidine,
4-[N-(benzyl)amino]-2,2,6,6-tetramethylpiperidine,
4-[N-(cyclohexyl)amino]-2,2,6,6-tetramethylpiperidine,
4-amino-2,6-diethyl-2,3,6-trimethylpiperidine, and bis-(2,2,6,6-tetramethyl-4-piperidinyl)amine,

Examples of suitable hydrazines include hydrazine, hydrazine hydrate, 35-85% hydrazine hydrate, methyl, ethyl, propyl,, isopropyl, n-butyl, sec-butyl, n-amyl, sec-amyl, n-hexyl and n- and sec-octyl hydrazines.

Preferably, the amine is 4-amino-2,2,6,6-tetramethylpiperidine or bis-(2,2,6,6-tetramethyl-4-piperidinyl)amine, the acid chloride is ethyl (or methyl)oxalyl chloride or ethyl (or methyl) succinyl chloride and the hydrazine is 85% hydrazine hydrate.

The reaction sequence of Method B is illustrated by the following equations:

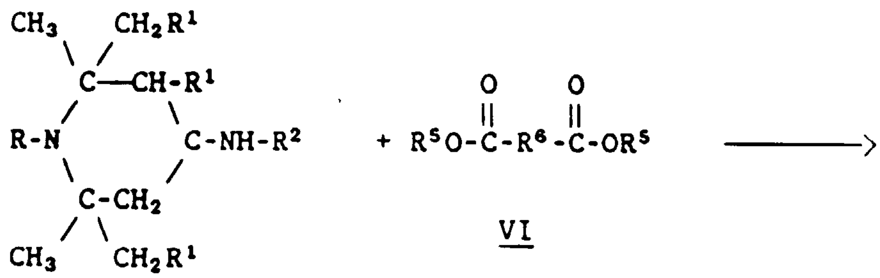

II + VI

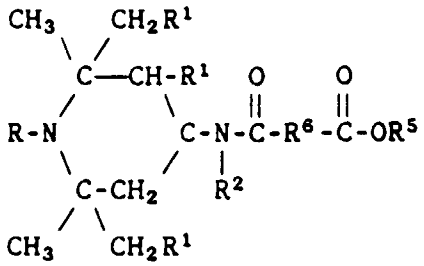

VII

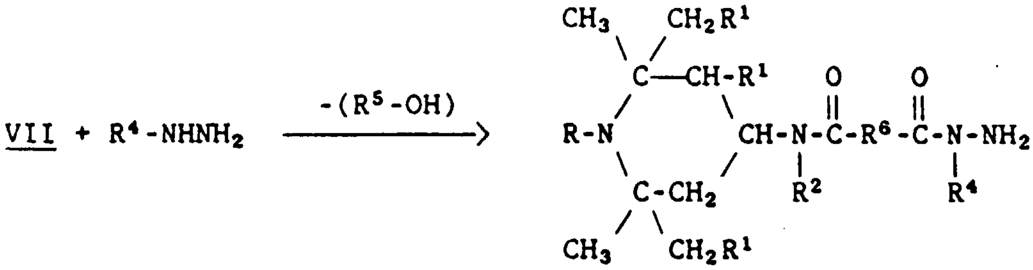


VIII

where $R^6$ is the same as $R^3$ except $R^6$ may not be an alkenylene diradical or an ethylene diradical.

The reactions are preferably run neat, or in alcoholic or glycol solvents and most preferably neat or in methanol if the diesters are activated. The amines (II) react quite readily with the oxalic acid diesters at room temperature and gentle cooling is advisable in the early stages of the reaction to minimize side reactions. In the case of the other diesters (i.e, $R^6$ is not a direct bond), heating or refluxing is necessary. The reactions can be monitored by gas chromatography and depending upon the starting amines and diesters, the reaction temperature can be adjusted up or down to speed up or slow down the reaction. In some cases it is preferable to distill of the alcohol as it forms.

When using dialkyl oxalates in methanol or ethanol, it is preferable to cool the alcohol solution of the oxalate below room temperature (5-10°C) before adding the amine and then let the temperature rise to room temperature or above over the course of 1/2 to 1 hour. After the oxalate is converted to the half ester-half amide, an equivalent amount of a primary or secondary alkyl hydrazine, hydrazine or hydrazine hydrate is added. The (alkyl) hydrazine readily reacts with the half-ester to form the amide-hydrazide. Any unreacted dialkyl oxalate is readily converted to insoluble oxalic acid dihydrazide which is removed by filtration. Depending upon the amount of alcohol present, the reaction mixture may have to be heated to dissolve all the product before filtering off the oxalic acid dihydrazide. The product is isolated by stripping off the solvent or by cooling the filtrate and crystallizing out the product.

When using dialkyl oxalates, the first step of the reaction sequence is preferably run in excess dialkyl oxalate to minimize side products. The excess oxalate is removed, preferably by vacuum stripping, and the hydrazinolysis of the intermediate half ester-half amide is preferably carried out in methanol, ethanol, propanol, or isopropanol.

When using diesters of other dicarboxylic acids, more severe heating conditions are required for both steps. However, the reactions can be monitored by infrared spectroscopy, liquid or gas chromatography and the heating conditions and length of reaction adjusted accordingly.

Suitable amines (II) are those illustrated for Method A. Likewise the same hydrazines are suitable for both Methods A and B. Suitable diesters include the methyl, ethyl, propyl, isopropyl and phenyl diesters or mixtures thereof of oxalic, malonic, substituted malonic, glutaric, adipic, 2-methylglutaric, 3-methylglutaric, 2,2-dimethylglutaric, 3,3-dimethylglutaric, pimelic, adipic, suberic, azelaic, sebacic, undecanedioic, 1,10-decanedicarboxylic, 1,12-dodecanedicarboxylic, o-, m- and p- phthalic and 3,3′-thiodipropionic acid. Diesters of fumaric acid and succinic acid were found to be unacceptable in this method.

Preferably, the amine is 4-amino-2,2,6,6-tetramethylpiperidine, the diester is diethyl oxalate, and the hydrazine is 85% hydrazine hydrate; the first step is run in excess diethyl oxalate and the hydrazinolysis step is run in methanol.

Most of the amines (II) can be prepared by reductive amination of triacetonamine (or its alkylated analogs) or by alkylation or acylation of triacetonamine followed by reductive amination (See U.S. Patent 4,191,683 or W. B. Lutz, S. Lazarus and R. I. Meltzer, J. Org. Chem 1962, 27, 1695). The alkylated and acylated derivatives (i.e, R is alkyl, alkenyl, aralkyl, aliphatic acyl, aromatic acyl, alkoxycarbonyl, aryloxycarbonyl, alkyl, aryl, cycloalkyl or aralkyl substituted carbamoyl, 2-cyanoethyl, or hydroxyalkyl) can be prepared by alkylating or acylating the unsubtituted (i.e, R is H) intermediate half ester-half amide by techniques well known in the art (e.g, acylation with acid chlorides, chloroformates,

carbamoyl chlorides, isocyanates etc. or alkylation with alkyl halides or epoxides) (See U.S. Pats. 4,348,524 and 4,191,683) followed by hydrazinolysis in a polar solvent. The mono esters of the diacid chlorides are either commercially available or can be readily prepared by methods well-known in the art from the corresponding mono esters of the dicarboxylic acids and chlorinating agents such as thionyl chloride, phosgene, phosphorous trichloride or phosphorous pentachloride.

The following examples are intended as illustrations and not limitations of the invention.

Example I

Preparation of N-(2,2,6,6-tetramethyl-4-piperidinyl)-N′-aminosuccinamide

Into a 3-neck 500 ml round bottom flask were weighed 31.5 grams (0.2 mole) of 4-amino-2,2,6,6-tetramethylpiperidine and 30.3 grams (0.3 mole) of triethylamine. The amines were diluted with 200 ml methylene chloride and the flask was equipped with a magnetic stirrer, thermometer, reflux condenser, and dropping funnel containing 32.9 grams (0.2 mole) ethyl succinyl chloride. The flask and its contents were cooled in an ice bath to 5°C and the acid chloride was added dropwise to the stirring solution over 1/2 hour while holding the temperature between 5-15°C. After the addition was completed, the ice bath was removed and the solution was stirred an additional hour. The $CH_2Cl_2$ solution was washed three times with 100 ml portions of water, dried over anhydrous $Na_2SO_4$, filtered and the methylene chloride stripped off on a rotating evaporator under reduced pressure. The residue was an orange-yellow viscous liquid weighing 38.2 grams. An infrared scan of the residue showed a strong NH band at 3300 $cm^{-1}$ and two strong carbonyl peaks at 1730 $cm^{-1}$ and 1640 $cm^{-1}$ and an amide band at 1550 $cm^{-1}$.

The residue was dissolved in 200 ml of ethanol and transferred to a 500 ml 3-neck flask equipped with a magnetic stirrer, thermometer and dropping funnel containing 26.4 grams of 85% hydrazine hydrate. The hydrazine hydrate was added dropwise over 5 minutes at room temperature. The reaction was stirred 1 hour at room temperature and then allowed to stand overnight. An IR scan indicated there was a small amount of ester unreacted (shoulder at 1730 $cm^{-1}$) so the reaction mixture was refluxed for 1 hour. The ethanol, water, and residual hydrazine were stripped off on a rotating evaporator under vacuum leaving a mushy solid. The solids were slurried in methylene chloride, were filtered off, and were air dried. The dry product weighed 28.2 grams. It was purified by recrystallization from isopropanol. The purified product melted at 208-210°C. The infrared spectra of the product (nujol mull) contained a very sharp NH peak at 3300 $cm^{-1}$ and a broad NH band centered at 3230 $cm^{-1}$, a strong carbonyl band with shoulders centered at 1610 $cm^{-1}$ and an amide band at 1550 $cm^{-1}$. The ester band at 1730 $cm^{-1}$ had completely disappeared.

Example II

Preparation of N-(2,2,6,6-tetramethyl-4-piperidinyl)-N′-aminomalonamide

Into a 3-neck 250 ml round bottom flask were weighed 15.6 grams (0.1 mole) of 4-amino-2,2,6,6-tetramethylpiperidine and 10.1 grams (0.1 mole) of triethylamine. The amines were diluted with 100 ml of methylene chloride; the flask was equipped with a magnetic stirrer, thermometer, reflux condenser, and dropping funnel containing 11.8 grams (0.078 mole) of ethyl malonyl chloride. The ethyl malonyl chloride was added dropwise to the stirring solution of the amines without any cooling. The reaction was quite exothermic and the temperature rose from 22°C to 42°C where the refluxing methylene chloride controlled the temperature. The reaction was stirred for an additional hour while the temperature receded to 35°C. The clear solution was allowed to stand overnight. The next day the reaction mixture was added to a stirring solution of 10.6 grams of sodium carbonate in 200 ml of water. The methylene chloride layer was separated and stripped off on a rotating evaporator. The residue weighed 17.9 grams. An infrared scan of the residue showed a strong carbonyl at 1740 $cm^{-1}$ (ester), a carbonyl at 1660-1680 $cm^{-1}$ (amide), and an amide band at 1550 $cm^{-1}$. An additional 1.1 grams of intermediate was obtained by reextracting the aqueous layer with another 100 ml of methylene chloride and stripping off the solvent.

The intermediate was dissolved in 100 ml of ethanol in a 250 ml 3-neck flask equipped with a reflux condenser, a thermometer, a magnetic stirrer, and a dropping funnel containing 11.8 grams (0.2 mole) of 85% hydrazine hydrate. The hydrazine hydrate was added over 5 minutes and then the reaction was stirred an additional 1-1/4 hours at room temperature, warmed to reflux, and refluxed 2 hours. (Gas chromatography indicated the hydrazinolysis was completed after the 1 hour stir at room temperature). The reaction was cooled to 60°C and filtered through a fluted filter to remove a small amount of insolubles. The filtrate was then stripped to dryness on a rotating evaporator under vacuum. The residue weighed 18.8 grams and was pink in color. The color seemed to carry over from the starting ethyl malonyl chloride. The crude product was recrystallized from isopropanol. The infrared spectra of the product (in methylene chloride) showed a broad NH band at 3300 $cm^{-1}$, a strong carbonyl band with shoulders centered at 1680 $cm^{-1}$, and an amide band at 1550 $cm^{-1}$. The ester band at 1740 $cm^{-1}$ had completely disappeared. The recrystallized product

had a melting range of 177-179°C.

Example III

Preparation of N-(2,2,6,6-tetramethyl-4-piperidinyl)-N′-aminooxamide

Method A

Into a 3-neck 500 ml round bottom flask was weighed 32.0 g (0.2 mole) of 4-amino-2,2,6,6-tetramethylpiperidine. The amine was diluted with 200 ml of methylene chloride and the flask was equipped as in Example I. The dropping funnel contained a solution of 13.7 grams (0.1 mole) of ethyl oxalyl chloride in 15 ml of methylene chloride. The acid chloride solution was added to the stirring amine solution over 1/2 hour while holding the temperature between 10-20°C. The reaction was stirred an additional 2 hours at room temperature. The reaction mixture was washed twice with 50 ml of water and twice with 50 ml of saturated sodium bicarbonate solution, was dried over anhydrous $Na_2SO_4$, and was filtered; the methylene chloride was stripped off on a rotating evaporator under reduced pressure. The residue was a light yellow viscous liquid which slowly solidified. The residue weighed 19.8 grams (77% crude yield). An infrared spectrum of the residue showed an NH band at 3300 $cm^{-1}$, strong carbonyl bands at 1730 $cm^{-1}$ (ester) and 1680 $cm^{-1}$ (amide), and an amide band at 1530 $cm^{-1}$.

The residue was dissolved in 100 ml of ethanol in a 250 ml 3-neck flask equipped as in Example I. The dropping funnel contained 13.4 grams (0.225 mole) of 85% hydrazine hydrate which was added over 20 minutes to the stirring ethanol solution at 30°C. The reaction was stirred an additional hour. The reaction mixture was transferred to a round bottom flask and the ethanol and excess hydrazine were stripped off on a rotating evaporator leaving 21.5 grams of a light yellow viscous liquid which solidified to a white waxy solid upon standing. The crude product contained a small amount of residual solvent. The product was recrystallized from isopropanol yielding a white powder having a melting point of 153-155°C. The infrared spectra of the product showed NH bands at 3300 $cm^{-1}$, a strong carbonyl band with shoulders centered at 1640-1670 $cm^{-1}$, a weak carbonyl band at 1600 $cm^{-1}$, and an amide band at 1530 $cm^{-1}$. The ester band at 1730 $cm^{-1}$ had completely disappeared (nujol mull). In chloroform solution the infrared spectrum had NH bands at 3300 and 3400 $cm^{-1}$, a very strong carbonyl band at 1680 $cm^{-1}$ and another carbonyl band at 1620 $cm^{-1}$ and an amide band at 1510 $cm^{-1}$.

Method B

Into a 3-neck 500 ml round bottom flask was weighed 43.8 grams (0.3 mole) of diethyl oxalate. The diethyl oxalate was diluted with 280 ml of methanol and cooled to 5°C in an ice water bath. The flask was equipped with a magnetic stirrer, a thermometer, a reflux condenser and an addition funnel containing 47.0 grams (0.3 mole) of 4-amino-2,2,6,6-tetramethylpiperidine. The amine was added to the stirred solution over 40 minutes at 5°C and for 1-1/4 hours at 20°C. Upon completion of the stirring periods the rection mixture was cooled to 15°C and 17-1/2 ml (0.3 mole) of 85% hydrazine hydrate were added dropwise over approximately 1 hour. The first 12 ml were added over 20 minutes at 15-22°C. The remainder was added in increments over 50 minutes while following the reaction by gas chromatography. Upon disappearance of the intermediate oxamate, the reaction mixture was heated to reflux to dissolve any insoluble product. The reaction was filtered hot to remove the insoluble oxalic acid dihydrazide (side product). The filtrate was cooled to 10°C and the white solids that formed were filtered off and vacuum dried for 2 hours at 50°C. The product weighed 48.8 grams and assayed 98.4% by gas chromatographic analysis. The infrared spectra was similar to the infrared spectra of the product obtained by Method A.

The methanol filtrate was stripped to dryness leaving 16.3 grams of a mixture containing approximately 58% of the desired product and 34% N,N′-bis(2,2,6,6-tetramethyl-4-piperidinyl)oxamide as analyzed by gas chromatography.

Alternate Method B

Step 1.

Into a 3-neck 1500 ml round bottom flask was added 730.5 grams (5.0 mole) of diethyl oxalate. The flask was equipped with a magnetic stirring bar, a thermometer, and a dropping funnel containing 156.3 grams (1.0 mole) 4-amino-2,2,6,6-tetramethylpiperidine. The amine was added dropwise to the stirring diethyl oxalate over 15 minutes while controlling the temperatures between 20 and 30°C. The reaction mixture was stirred an additional 15 minutes at room temperature. The magnetic stirrer, thermometer, and addition funnel were removed and the side necks of the flask stoppered. The flask was then placed on a rotating evaporator and the ethanol generated during the reaction was stripped off under reduced pressure at 25-35°C. After the ethanol had been stripped off, the vacuum was released and

the receiver drained of ethanol. The vacuum was reapplied and the excess diethyl oxalate stripped off by heating the flask in an oil bath at 140°C under full vacuum. After constant weight was reached, the residue was cooled to room temperature, dissolved in 200 ml of methanol, and transferred to a 500 ml dropping funnel.

Step 2.

Into a 3-neck 1 liter flask containing a magnetic stirrer, a thermometer, and the addition funnel containing the methanol solution of ethyl N-(2,2,6,6-tetramethyl-4-piperidyl)oxamate from Step 1, were added 400 ml of methanol followed by 67 grams (1.14 moles) of 85% hydrazine hydrate. The temperature of the solution was adjusted to 10°C with a water bath. The methanol solution of the intermediate was added to the stirred hydrazine solution dropwise over 15 minutes while controlling the temperature at 10-20°C. After the addition was completed, the reaction mixture was stirred an additional 20 minutes at room temperature and then warmed to 60-65°C to dissolve the product in the methanol. The warm solution was stirred 5 minutes at 60-65°C and then filtered warm to remove any insoluble oxalic acid dihydrazide. The filtrate was cooled with stirring to 0°C to crystallize the product.

The crystallized product was filtered, washed with hexane, refiltered, and dried overnight in a vacuum oven at 65°C. The dry product weighed 218.5 grams and assayed 98%.

Example IV

Preparation of N-(2,2,6,6-tetramethyl-4-piperidinyl)-N′-aminoadipamide

Method A

Adipoyl chloride monomethyl ester was prepared from adipic acid monomethyl ester and thionyl chloride.

Into a 3-neck 500 ml round bottom flask was weighed 34.4 grams (0.22 mole) of 4-amino-2,2,6,6-tetramethylpiperidine. The amine was diluted with 200 ml methylene chloride; the flask was equipped as in Example I. The dropping funnel contained 17.9 grams (0.1 mole) of adipoyl chloride monomethyl ester. The acid chloride was added to the stirring amine solution over 10 minutes while holding the temperature below 20°C with an ice-water bath. The reaction flask was stirred an additional 30 minutes at room temperature and then the contents in the flask were added to a solution of 10.6 grams (0.1 mole) of sodium carbonate in 200 ml of water. The mixture was stirred for 5 minutes and then was transferred to a separatory funnel. The methylene chloride layer was separated, washed with 200 ml of water, dried over anhydrous sodium sulfate, and filtered; the methylene chloride was stripped off on a rotating evaporator under reduced pressure. The residue was a light yellow viscous liquid weighing 25.5 grams. An infrared spectrum of the intermediate product showed an NH band at 3300 $cm^{-1}$, strong carbonyl bands at 1740 $cm^{-1}$ (ester) and 1650 $cm^{-1}$ (amide), and an amide band at 1560 $cm^{-1}$.

The residue was dissolved in 200 ml of methanol and transferred to a 500 ml 3-neck flask equipped with a thermometer, magnetic stirrer, reflux condenser, and dropping funnel containing 15.2 grams (0.225 mole) of 85% hydrazine hydrate. The hydrazine hydrate was added dropwise over 10 minutes at 27-28°C. The reaction mixture was heated to 50°C in a water bath and stirred 1 hour at 45-50°C. It was then allowed to stand at room temperature over the weekend. A gas chromatographic scan indicated all the intermediate adipamate had reacted. The reaction mixture was transferred to a round bottom flask and the methanol, water, and excess hydrazine were stripped off on a rotating evaporator leaving a white solid. The solid was slurried in hexane, filtered, and air dried. The product weighed 23.65 grams and had a melting point of 170-173°C. The infrared spectra of the product (nujol mull) showed NH bands at 3240 $cm^{-1}$, strong carbonyls at 1600 $cm^{-1}$, and an amide band at 1550 $cm^{-1}$. The ester band at 1740 $cm^{-1}$ had completely disappeared. In chloroform solution the infrared spectra showed NH bands at 3240 $cm^{-1}$, 3340 $cm^{-1}$, and 3440 $cm^{-1}$, a strong carbonyl band at 1650 $cm^{-1}$, and an amide band 1510 $cm^{-1}$.

Method B

Into a 50 ml 3-neck flask were weighed 17.4 grams (0.1 mole) of dimethyl adipate and 15.6 grams (0.1 mole) of 4-amino-2,2,6,6-tetramethylpiperidine. The flask was equipped with a magnetic stirring bar, thermometer, Dean-Stark trap, and reflux condenser. The flask was placed in an oil bath and heated to 166°C over 1-1/2 hours and heated an additional 4 hours with the temperature rising to 173°C; 3.0 ml of methanol formed in the Dean-Stark trap. The reaction was cooled to room temperature and analyzed by gas chromatography and liquid chromatography. The semi-solid product was a mixture of the starting dimethyl adipate, the desired methyl N-(2,2,6,6-tetramethyl-4-piperidinyl)adipamate and N,N′-di(2,2,6,6-tetramethyl-4-piperidinyl)adipamide. The 4-amino-2,2,6,6-tetramethylpiperidene had completely reacted.

The semi-solid mixture was slurried in 100 ml of methyl t-butyl ether and filtered to remove the diamide impurity

(8.9 g). The filtrate was stripped on a rotating evaporator to remove the methyl t-butyl ether. The residue was a yellow viscous liquid (13.6 g). Gas chromatography indicated it was a mixture of approximately 70% of the desired adipamate and 25-30% dimethyl adipate.

Into a 100 ml 3-neck flask were added the crude mixture of the methyl N-(2,2,6,6-tetramethyl-4-piperidinyl)adipamate and dimethyl adipate and 50 ml of methanol. The flask was equipped with a magnetic stirrer, thermometer, reflux condenser and addition funnel containing 4.5 g (0.077 mole) of 85% hydrazine hydrate. The hydrazine hydrate was added dropwise over 5 minutes and the reaction mixture was stirred an additional 1-1/2 hours at room temperature, heated to reflux, and refluxed 11 hours at 68°C filtered hot. The filtrate was cooled to room temperature and refiltered. The combined filter cake after drying weighed 2.8 grams and was primarily adipic acid dihydrazide. The filtrate was stripped on a rotating evaporator leaving 8.5 grams of a cream colored solid. The product contained approximately 1.4% residual adipic acid dihydrazide. An infrared scan of the product (chloroform solution) showed a sharp NH band at 3440 $cm^{-1}$ and a broad NH band at 3300 $cm^{-1}$, a very strong carbonyl band at 1650 $cm^{-1}$, and an amide band at 1510 $cm^{-1}$. A wet method assay indicated the product was 80% N-(2,2,6,6-tetramethyl-4-piperidinyl)-N′-aminooxamide.

Example V

Preparation of N-(2,2,6,6-tetramethyl-4-piperidinyl)-N′-aminoazelamide

Method A

Azelaoyl chloride monomethyl ester was prepared from azelaic acid monomethyl ester and thionyl chloride.

Methyl N-(2,2,6,6-tetramethyl-4-piperidinyl)azelamate was prepared in the same manner as the preparation of methyl N- (2,2,6,6-tetramethyl-4-piperidinyl)adipamate (Example IV) except 23.16 grams (0.1 mole) of azelaoyl chloride monomethyl ester was substituted for the adipoyl chloride monomethyl ester used in Example IV. The product was a light yellow viscous liquid weighing 32.1 grams. An infrared spectrum of the intermediate product showed an NH band at 3300 $cm^{-1}$, a strong carbonyl band at 1760 $cm^{-1}$ (ester), a strong broad carbonyl band at 1640-1660 $cm^{-1}$, and an amide band at 1550 $cm^{-1}$.

The intermediate product was dissolved in 200 ml methanol and treated with 15.2 grams (0.225 mole) of 85% hydrazine hydrate as in Example IV. After stirring for two days at room temperature, a gas chromatographic scan indicated that all of the azelamate had reacted. The reaction mixture was transferred to a 500 ml round bottom flask and the methanol, water, and excess hydrazine hydrate were stripped off. The residue was a pasty solid. It was slurried in methylene chloride and the solids were filtered off. The solids were transferred to a flask to strip off any residual methylene chloride. The flask was heated with a heat gun and the solids were melted and resolidified upon cooling. The product was a sticky solid which weighed 24.7 grams.

The infrared spectra of the product (chloroform solution) showed an NH band at 3300 $cm^{-1}$, a strong carbonyl band centered at 1640 $cm^{-1}$, and an amide band at 1520-1540 $cm^{-1}$. The ester band at 1760 $cm^{-1}$ had completely disappeared.

Example VI

Preparation of N-(1-acetyl-2,2,6,6-tetramethyl-4-piperidinyl)-N′-aminooxamide

Methyl N-(2,2,6,6-tetramethyl-4-piperidinyl)oxamate was prepared from diethyloxalate and 4-amino-2,2,6,6-tetramethylpiperidine according to the first step in Example IIIB. The ethyl ester undergoes ester interchange in methanol to form the methyl ester.

Into a 125 ml 3-neck flask was added 18.2 grams (0.075 mole) of methyl N-(2,2,6,6-tetramethyl-4-piperidinyl) oxamate, 75 ml of methylene chloride and 8.1 grams (0.08 mole) of triethylamine. The flask was equipped with a magnetic stirrer, thermometer, reflux condenser, and addition funnel containing 5.9 grams (0.075 mole) of acetyl chloride in 10 ml of methylene chloride. The solution in the flask was cooled to 7°C in an ice bath and the acetyl chloride solution was added dropwise while holding the temperature at 7-9°C. The addition was exothermic and white solids formed during the addition. The reaction mixture was stirred 1 hour at 5-15°C. Gas chromatography indicated that the starting material had disappeared and a new higher boiling material had formed. The reaction mixture was stirred into 100 ml of saturated sodium bicarbonate solution. The dark methylene chloride layer was separated and washed with an additional 50 ml of saturated sodium bicarbonate solution. The methylene chloride layer was separated, dried over anhydrous sodium sulfate, filtered, and stripped off on a rotating evaporator. The brown residue (14.5 grams) was slurried in methyl t-butyl ether and filtered. The tan filter cake was air dried and weighed 11.5 grams. The product had a melting point of 125-129°C. An infrared scan (chloroform solution) contained strong carbonyl bands at 1675 $cm^{-1}$, 1600 $cm^{-1}$, and 1495 $cm^{-1}$, a weak carbonyl band at 1715 $cm^{-1}$, and a weak shoulder at 1745 $cm^{-1}$.

Into a 100 ml 3-neck flask was weighed 8.0 grams (0.028 mole) of methyl N-(1-acetyl-2,2,6,6-tetramethyl-4-piperidinyl)-oxamate (prepared above); 50 ml of methanol was added. The flask was equipped with a magnetic stirrer, thermometer, reflux condenser, and a dropping funnel containing 1.7 ml of (0.029 mole) of 85% hydrazine hydrate. The 85% hydrazine hydrate was added dropwise to the stirring slurry of the acetylated oxamate. The reaction temperature rose from 20 to 26°C during the addition. The reaction was stirred for an additional hour at room temperature. A gas chromatographic scan taken 45 minutes into the stir period indicated that all of the starting oxamate had reacted to form the hydrazide. The reaction slurry was filtered and the white filter cake was air dried. A gas chromatographic scan of a methylene chloride solution of the product indicated the complete absence of the starting oxamate and the presence of a new higher boiling peak. A liquid chromatographic scan showed the presence of a single component. An infrared scan of the product in chloroform solution had strong carbonyl bands at 1660 $cm^{-1}$, 1610 $cm^{-1}$, and 1490 $cm^{-1}$. The dried product weighed 5.0 grams (m.p. 126-129°C).

## Claims

**Claims for the following Contracting States : BE, CH, DE, FR, GB, IT, LI, NL, SE**

1. A compound of the formula

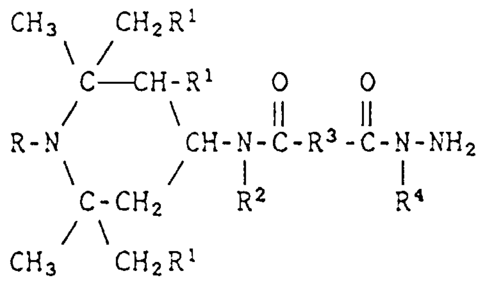

wherein:

R is selected from hydrogen, oxyl, hydroxyl, alkyl of 1 to 20 carbons, alkenyl or alkynyl of 3 to 8 carbons, aralkyl of 7 to 12 carbons, aliphatic acyl of 1 to 10 carbons, aromatic acyl of 7 to 13 carbons, alkoxycarbonyl of 2 to 9 carbons, aryloxycarbonyl of 7 to 15 carbons, alkyl, aryl, cycloalkyl or aralkyl substituted carbamoyl of 2 to 13 carbons, 2-cyanoethyl, hydroxyalkyl of 1 to 6 carbons, epoxyalkyl of 3 to 10 carbons, or a polyalkylene oxide of 4 to 30 carbons,

$R^1$ is selected from hydrogen or lower alkyl of 1 to 4 carbons,

$R^2$ is selected from hydrogen, alkyl of 1 to 10 carbons, cycloalkyl of 5 to 12 carbons, aralkyl of 7 to 12 carbons, aryl of 6 to 12 carbons, 2-cyanoethyl, or a radical of the formula

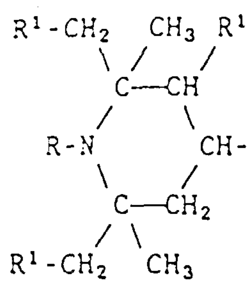

$R^3$ is selected from a direct bond, alkylene or branched alkylene diradical of 1 to 14 carbons, alkenylene diradical of 2 to 10 carbons, an oxydialkylene or thiodialkylene diradical of 4 to 10 carbons or o-, m- or p-phenylene or substituted phenylene diradical where the substituent may be $C_1$-$C_4$ alkyl, $C_1$-$C_4$ alkoxy, hydroxy, bromine, chlorine, mercapto, or $C_1$-$C_4$ alkyl mercapto, and

$R^4$ is selected from hydrogen, a primary or secondary alkyl group of 1 to 8 carbons, an aralkyl group of 7 to 12 carbons, or a cycloalkyl group of 5 to 12 carbons.

2. The compound of claim 1 where R is selected from hydrogen, alkyl of 1 to 4 carbons, alkenyl of 3 carbons, benzyl,

2-cyanoethyl, or acetyl,

$R^1$ is selected from hydrogen or methyl,
$R^2$ is selected from hydrogen, alkyl of 1 to 4 carbons, or 2,2,6,6-tetramethyl-4-piperidinyl,
$R^3$ is selected from a direct bond, an alkylene diradical of 1 to 8 carbons, or an o-, m- or p-phenylene diradical, and
$R^4$ is selected from hydrogen, primary or secondary alkyl of 1 to 4 carbons, benzyl, or cyclohexyl.

**3.** The compound of claim 2 where

R is selected from hydrogen, methyl, or acetyl,
$R^1$ is hydrogen,
$R^2$ is selected from hydrogen or 2,2,6,6-tetramethyl-4-piperidinyl,
$R^3$ is selected from a direct bond or a 1,2 ethylene diradical, and
$R^4$ is hydrogen or methyl.

**4.** The compound of claim 3 where
R, $R^1$, $R^2$ and $R^4$ are hydrogen and $R^3$ is a direct bond or an alkylene diradical of 1-8 carbons, especially a 1,2-ethylene diradical.

**5.** The compound of claim 3 where $R^1$, $R^2$ and $R^4$ are hydrogen, R is methyl or acetyl and $R^3$ is a direct bond.

**6.** A process for preparing the compound of claim 1 by (1) reacting an amine of the formula

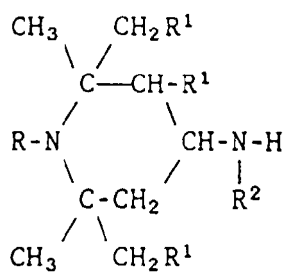
,

with a mono ester acid chloride of the formula

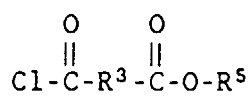

in an inert solvent such as a hydrocarbon, chlorinated hydrocarbon or ether solvent, and then (2) reacting the intermediate half ester-half amide

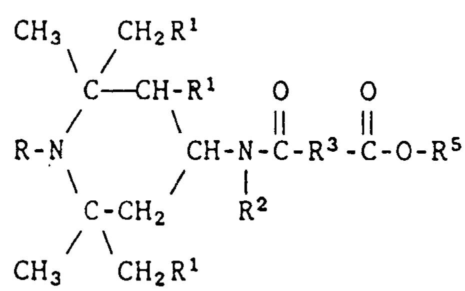

with a hydrazine of the formula $R^4$-NH-$NH_2$ in a polar solvent and isolating the compound of claim 1 by crystallization from the polar solvent or by evaporation of the solvent where R, $R^1$, $R^2$, $R^3$, and $R^4$ are as previously defined in claim 1 and $R^5$ is lower alkyl of 1 to 6 carbons or phenyl.

**7.** The process of claim 6 where R, $R^1$, $R^2$, $R^3$ and $R^4$ are as previously defined in claim 2, $R^5$ is methyl or ethyl, the solvent in step (1) is a hydrocarbon or chlorinated hydrocarbon solvent and the solvent in step (2) is a low molecular

weight alcohol or glycol.

**8.** The process of claim 7 where $R^1$, $R^2$, $R^3$ and $R^4$ are as previously defined in claim 3 and $R^5$ is methyl or ethyl.

**9.** The process of claim 7 where the amine is 4-amino-2,2, 6,6-tetramethylpiperidine, the mono ester acid chloride is ethyl oxalyl chloride or ethyl succinyl chloride, the hydrazine is 85% hydrazine hydrate, the solvent in step (1) is methylene chloride and the solvent in step (2) is methanol.

**10.** A process for the preparation of the compound of claim 1 having structure

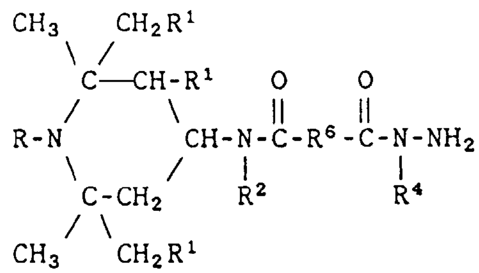

where R, $R^1$,$R^2$ and $R^4$ are as previously defined in claim 1 and $R^6$ is a direct bond, an alkylene or branched alkylene diradical of one carbon or 3 to 14 carbons, an oxydialkylene or thiodialkylene diradical of 4 to 10 carbons or an o-, m- or p-phenylene or substituted phenylene diradical where the substituents may be $C_1$-$C_4$ alkyl, $C_1$-$C_4$ alkoxy, hydroxy, bromine, chlorine, mercapto or $C_1$-$C_4$ alkylmercapto, by (1) reacting an amine of formula

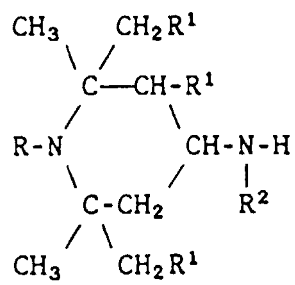

with a dialkyl or diphenyl diester of formula

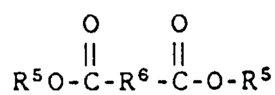

either neat or in the presence of a polar solvent to form an intermediate half ester-half amide of formula

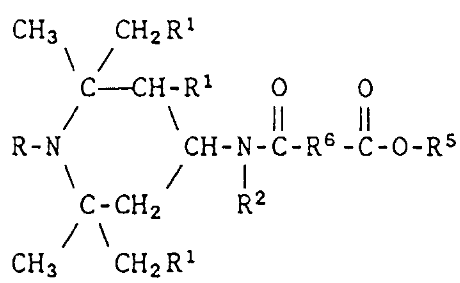

and then (2) reacting the intermediate with a hydrazine of formula $R^4NH-NH_2$ and isolating a product of formula

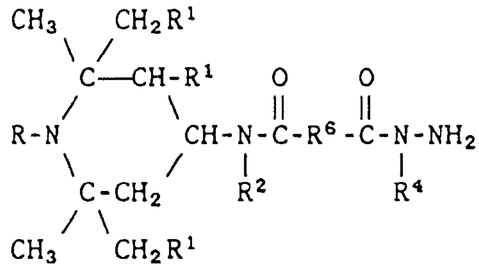

by crystallization from the solvent or by evaporation of the solvent where R, $R^1$, $R^2$, $R^4$ and $R^6$ are as previously defined and $R^5$ is as defined in claim 6.

**11.** The process of claim 10 where the amine is reacted with a dialkyl or diphenyl oxalate where $R^6$ is a direct bond, neat or in a polar solvent and then reacting the intermediate half-ester half-amide where $R^6$ is a direct bond with a hydrazine in a polar solvent and isolating the compound of claim 1 where $R^3$ is a direct bond by crystallization from the solvent or by evaporation of the solvent.

**12.** The process of claim 10 where the amine is 4-amino-2,2,6,6-tetramethylpiperidine, the diester is dimethyl adipate, the hydrazine is hydrazine hydrate and step (1) is run neat and the hydrazinolysis is run in methanol, ethanol, propanol or isopropanol.

**13.** A process of claim 11 where the amine is 4-amino-2,2,6,6-tetramethylpiperidine or bis-(2,2,6,6-tetramethyl-4-piperidinyl)amine, the oxalate diester is dimethyl or diethyl oxalate, the hydrazine is 85% hydrazine hydrate and the solvent is methanol, ethanol, propanol or isopropanol.

**14.** A process of claim 11 where the amine is 4-amino-2,2,6,6-tetramethylpiperidine, the oxalate diester is dimethyl or diethyl oxalate, the solvent is excess oxalate diester, the excess oxalate diester is removed and replaced with methanol or ethanol before reacting the intermediate with a hydrazine which is 85% hydrazine hydrate.

**15.** The use of a compound of any one of claims 1 to 5 for stabilizing polymers and copolymers.

**Claims for the following Contracting State : ES**

**1.** A process for preparing a compound of the formula

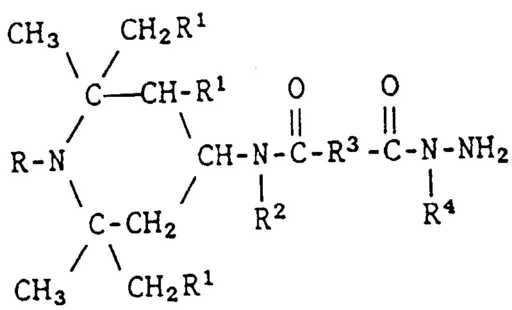

wherein:

R is selected from hydrogen, oxyl, hydroxyl, alkyl of 1 to 20 carbons, alkenyl or alkynyl of 3 to 8 carbons, aralkyl of 7 to 12 carbons, aliphatic acyl of 1 to 10 carbons, aromatic acyl of 7 to 13 carbons, alkoxycarbonyl of 2 to 9 carbons, aryloxycarbonyl of 7 to 15 carbons, alkyl, aryl, cycloalkyl or aralkyl substituted carbamoyl of 2 to 13 carbons, 2-cyanoethyl, hydroxyalkyl of 1 to 6 carbons, epoxyalkyl of 3 to 10 carbons, or a polyalkylene oxide of 4 to 30 carbons,
$R^1$ is selected from hydrogen or lower alkyl of 1 to 4 carbons,
$R^2$ is selected from hydrogen, alkyl of 1 to 10 carbons, cycloalkyl of 5 to 12 carbons, aralkyl of 7 to 12 carbons, aryl of 6 to 12 carbons, 2-cyanoethyl, or a radical of the formula

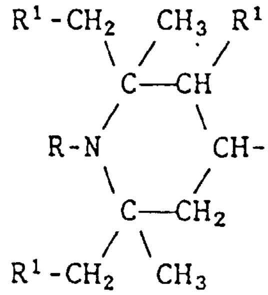

,

$R^3$ is selected from a direct bond, alkylene or branched alkylene diradical of 1 to 14 carbons, alkenylene diradical of 2 to 10 carbons, an oxydialkylene or thiodialkylene diradical of 4 to 10 carbons or o-, m- or p-phenylene or substituted phenylene diradical where the substituent may be $C_1$-$C_4$ alkyl, $C_1$-$C_4$ alkoxy, hydroxy, bromine, chlorine, mercapto, or $C_1$-$C_4$ alkyl mercapto, and
$R^4$ is selected from hydrogen, a primary or secondary alkyl group of 1 to 8 carbons, an aralkyl group of 7 to 12 carbons, or a cycloalkyl group of 5 to 12 carbons, by (1) reacting an amine of the formula

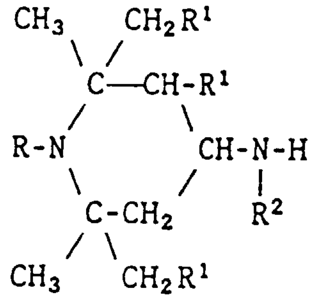

,

with a mono ester acid chloride of the formula

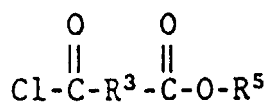

in an inert solvent such as a hydrocarbon, chlorinated hydrocarbon or ether solvent, and then (2) reacting the intermediate half ester-half amide

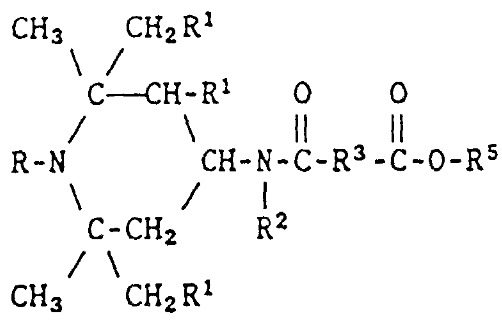

with a hydrazine of the formula $R^4$-NH-$NH_2$ in a polar solvent and isolating the compound by crystallization from the polar solvent or by evaporation of the solvent where R, $R^1$, $R^2$, $R^3$ and $R^4$ are as previously defined and $R^5$ is lower alkyl of 1 to 6 carbons or phenyl.

**2.** The process of claim 1 where R is selected from hydrogen, alkyl of 1 to 4 carbons, alkenyl of 3 carbons, benzyl, 2-cyanoethyl, or acetyl,

$R^1$ is selected from hydrogen or methyl,
$R^2$ is selected from hydrogen, alkyl of 1 to 4 carbons, or 2,2,6,6-tetramethyl-4-piperidinyl,
$R^3$ is selected from a direct bond, an alkylene diradical of 1 to 8 carbons, or an o-, m- or p-phenylene diradical, and

$R^4$ is selected from hydrogen, primary or secondary alkyl of 1 to 4 carbons, benzyl, or cyclohexyl, and
$R^5$ is methyl or ethyl, the solvent in step (1) is a hydrocarbon or chlorinated hydrocarbon solvent and the solvent in step (2) is a low molecular weight alcohol or glycol.

**3.** The process of claim 2 where

R is selected from hydrogen, methyl, or acetyl,
$R^1$ is hydrogen,
$R^2$ is selected from hydrogen or 2,2,6,6-tetramethyl-4-piperidinyl,
$R^3$ is selected from a direct bond or a 1,2-ethylene diradical, and
$R^4$ is hydrogen or methyl, and
$R^5$ is methyl or ethyl.

**4.** The process of claim 3 where
R, $R^1$, $R^2$, and $R^4$ are hydrogen, $R^3$ is a direct bond or an alkylene diradical of 1 to 8 carbons, especially a 1,2-ethylene diradical, $R^5$ is ethyl and the hydrazine is 85% hydrazine hydrate, the solvent in step (1) is methylene chloride and the solvent in step (2) is methanol.

**5.** The process of claim 3 where $R^1$, $R^2$ and $R^4$ are hydrogen, R is methyl or acetyl and $R^3$ is a direct bond.

**6.** A process of claim 1 for the preparation of a compound having structure

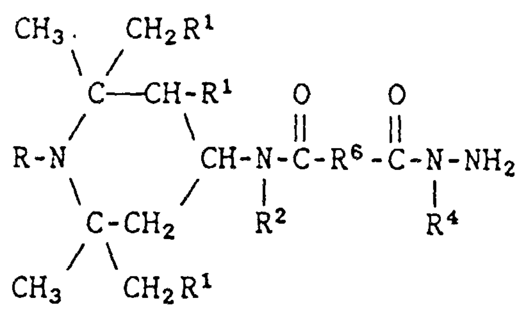

where R, $R^1$, $R^2$ and $R^4$ are as previously defined in claim 1 and $R^6$ is a direct bond, an alkylene or branched alkylene diradical of one carbon or 3 to 14 carbons, an oxydialkylene or thiodialkylene diradical of 4 to 10 carbons or an o-, m- or p-phenylene or substituted phenylene diradical where the substituents may be $C_1$-$C_4$ alkyl, $C_1$-$C_4$ alkoxy, hydroxy, bromine, chlorine, mercapto or $C_1$-$C_4$ alkylmercapto, by (1) reacting an amine of formula

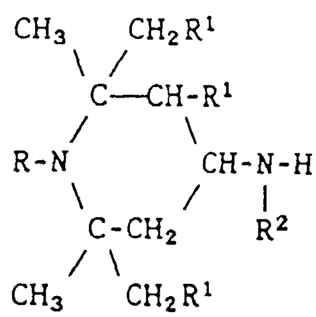

with a dialkyl or diphenyl diester of formula

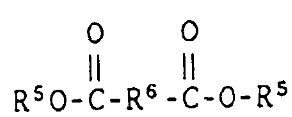

either neat or in the presence of a polar solvent to form an intermediate half ester-half amide of formula

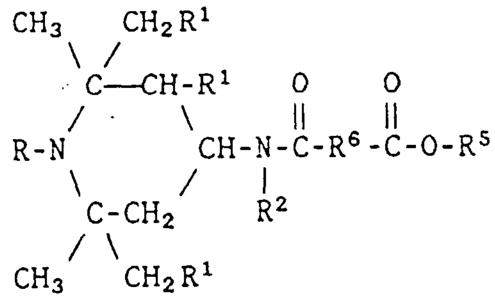

and then (2) reacting the intermediate with a hydrazine of formula $R^4NH\text{-}NH_2$ and isolating a product of formula

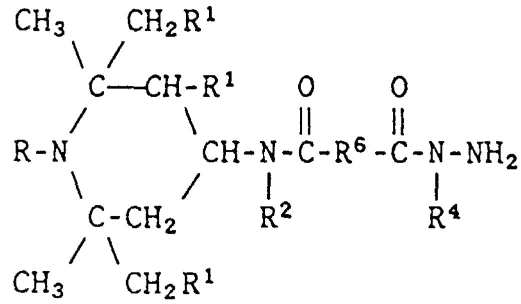

by crystallization from the solvent or by evaporation of the solvent where R, $R^1$, $R^2$, $R^4$ and $R^6$ are as previously defined and R is as defined in claim 1.

**7.** The process of claim 6 where the amine is reacted with a dialkyl or diphenyl oxalate where $R^6$ is a direct bond, neat or in a polar solvent and then reacting the intermediate half-ester half-amide where $R^6$ is a direct bond with a hydrazine in a polar solvent and isolating the compound of claim 1 where $R^3$ is a direct bond by crystallization from the solvent or by evaporation of the solvent.

**8.** The process of claim 6 where the amine is 4-amino-2,2,6,6-tetramethylpiperidine, the diester is dimethyl adipate, the hydrazine is hydrazine hydrate and step (1) is run neat and the hydrazinolysis is run in methanol, ethanol, propanol or isopropanol.

**9.** A process of claim 7 where the amine is 4-amino-2,2,6,6-tetramethylpiperidine or bis-(2,2,6,6-tetramethyl-4-piperidinyl)amine, the oxalate diester is dimethyl or diethyl oxalate, the hydrazine is 85% hydrazine hydrate and the solvent is methanol, ethanol, propanol or isopropanol.

**10.** A process of claim 7 where the amine is 4-amino-2,2,6,6-tetramethylpiperidine, the oxalate diester is dimethyl or diethyl oxalate, the solvent is excess oxalate diester, the excess oxalate diester is removed and replaced with methanol or ethanol before reacting the intermediate with a hydrazine which is 85% hydrazine hydrate.

**11.** The use of a compound prepared according to the processes of any one of claims 1 to 10 for stabilizing polymers and copolymers.

**Patentansprüche**

**Patentansprüche für folgende Vertragsstaaten : BE, CH, DE, FR, GB, IT, LI, NL, SE**

**1.** Eine Verbindung der Formel

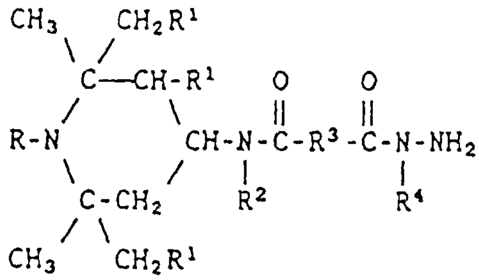

worin:

R ausgewählt ist aus Wasserstoff, Oxyl, Hydroxyl, Alkyl mit 1 bis 20 Kohlenstoffatomen, Alkenyl oder Alkinyl mit 3 bis 8 Kohlenstoffatomen, Aralkyl mit 7 bis 12 Kohlenstoffatomen, aliphatischem Acyl mit 1 bis 10 Kohlenstoffatomen, aromatischem Acyl mit 7 bis 13 Kohlenstoffatomen, Alkoxycarbonyl mit 2 bis 9 Kohlenstoffatomen, Aryloxycarbonyl mit 7 bis 15 Kohlenstoffatomen, alkyl-, aryl-, cycloalkyl- oder aralkylsubstituiertem Carbamoyl mit 2 bis 13 Kohlenstoffatomen, 2-Cyanoethyl, Hydroxyalkyl mit 1 bis 6 Kohlenstoffatomen, Epoxyalkyl mit 3 bis 10 Kohlenstoffatomen oder einem Polyalkylenoxid mit 4 bis 30 Kohlenstoffatomen,
$R^1$ ausgewählt ist aus Wasserstoff oder Niedrigalkyl mit 1 bis 4 Kohlenstoffatomen,
$R^2$ ausgewählt ist aus Wasserstoff, Alkyl mit 1 bis 10 Kohlenstoffatomen, Cycloalkyl mit 5 bis 12 Kohlenstoffatomen, Aralkyl mit 7 bis 12 Kohlenstoffatomen, Aryl mit 6 bis 12 Kohlenstoffatomen, 2-Cyanoethyl oder einem Rest der Formel

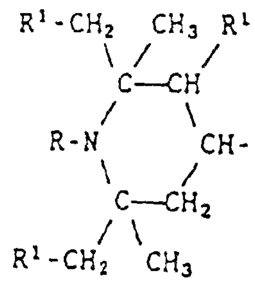

$R^3$ ausgewählt ist aus einer direkten Bindung, einem zweibindigen Alkylen- oder verzweigten zweibindigen Alkylenrest mit 1 bis 14 Kohlenstoffatomen, einem zweibindigen Alkenylenrest mit 2 bis 10 Kohlenstoffatomen, einem zweibindigen Oxydialkylen- oder zweibindigen Thiodialkylenrest mit 4 bis 10 Kohlenstoffatomen oder einem zweibindigen o-, m- oder p-Phenylen- oder substituierten zweibindigen Phenylenrest, wobei der Substituent $C_1$-$C_4$-Alkyl, $C_1$-$C_4$-Alkoxy, Hydroxy, Brom, Chlor, Mercapto oder $C_1$-$C_4$-Alkylmercapto sein kann, und
$R^4$ ausgewählt ist aus Wasserstoff, einer primären oder sekundären Alkylgruppe mit 1 bis 8 Kohlenstoffatomen, einer Aralkylgruppe mit 7 bis 12 Kohlenstoffatomen oder einer Cycloalkylgruppe mit 5 bis 12 Kohlenstoffatomen.

**2.** Die Verbindung nach Anspruch 1, worin R ausgewählt ist aus Wasserstoff, Alkyl mit 1 bis 4 Kohlenstoffatomen, Alkenyl mit 3 Kohlenstoffatomen, Benzyl, 2-Cyanoethyl oder Acetyl,

$R^1$ ausgewählt ist aus Wasserstoff oder Methyl,
$R^2$ ausgewählt ist aus Wasserstoff, Alkyl mit 1 bis 4
Kohlenstoffatomen oder 2,2,6,6-Tetramethyl-4-piperidinyl,
$R^3$ ausgewählt ist aus einer direkten Bindung, einem zweibindigen Alkylenrest mit 1 bis 8 Kohlenstoffatomen oder einem zweibindigen o-, m- oder p-Phenylenrest, und
$R^4$ ausgewählt ist aus Wasserstoff, primärem oder sekundärem Alkyl mit 1 bis 4 Kohlenstoffatomen, Benzyl oder Cyclohexyl.

**3.** Die Verbindung nach Anspruch 2, worin

R ausgewählt ist aus Wasserstoff, Methyl oder Acetyl,
$R^1$ Wasserstoff ist,
$R^2$ ausgewählt ist aus Wasserstoff oder 2,2,6,6-Tetramethyl-4-piperidinyl,

$R^3$ ausgewählt ist aus einer direkten Bindung oder einem zweibindigen 1,2-Ethylenrest, und
$R^4$ Wasserstoff oder Methyl ist.

**4.** Die Verbindung nach Anspruch 3, worin
R, $R^1$, $R^2$ und $R^4$ Wasserstoff sind, und $R^3$ eine direkte Bindung oder ein zweibindiger Alkylenrest mit 1-8 Kohlenstoffatomen ist, insbesondere ein zweibindiger 1,2-Ethylenrest.

**5.** Die Verbindung nach Anspruch 3, worin $R^1$, $R^2$ und $R^4$ Wasserstoff sind, R Methyl oder Acetyl ist, und $R^3$ eine direkte Bindung ist.

**6.** Ein Verfahren zur Herstellung der Verbindung nach Anspruch 1 durch (1) Umsetzung eines Amins der Formel

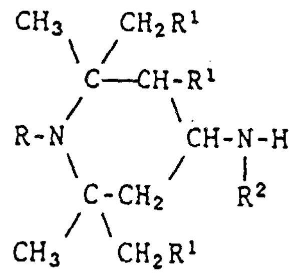

mit einem Monoester-Säurechlorid der Formel

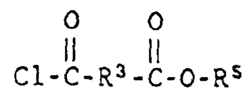

in einem inerten Lösungsmittel, wie z.B. einem Kohlenwasserstoff-, Chlorkohlenwasserstoff- oder Etherlösungsmittel, und anschließend (2) Umsetzung des Zwischenprodukts, das halb Ester halb Amid ist,

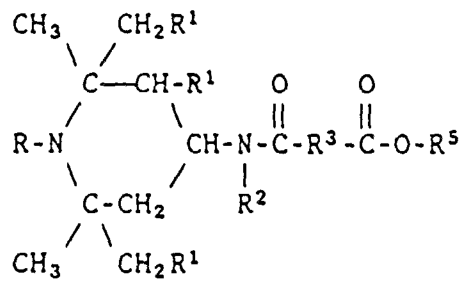

mit einem Hydrazin der Formel $R^4$-NH-$NH_2$ in einem polaren Lösungsmittel und Isolierung der Verbindung nach Anspruch 1 durch Kristallisation aus dem polaren Lösungsmittel oder durch Eindampfen des Lösungsmittels, wobei R, $R^1$, $R^2$, $R^3$ und $R^4$ wie zuvor in Anspruch 1 definiert sind, und $R^5$ Niedrigalkyl mit 1 bis 6 Kohlenstoffatomen oder Phenyl ist.

**7.** Das Verfahren nach Anspruch 6, worin R, $R^1$, $R^2$, $R^3$ und $R^4$ wie zuvor in Anspruch 2 definiert sind, $R^5$ Methyl oder Ethyl ist, das Lösungsmittel in Schritt (1) ein Kohlenwasserstoff- oder Chlorkohlenwasserstofflösungsmittel ist, und das Lösungsmittel in Schritt (2) ein Alkohol mit niedrigem Molekulargewicht oder Glycol ist.

**8.** Das Verfahren nach Anspruch 7, worin $R^1$, $R^2$, $R^3$ und $R^4$ wie zuvor in Anspruch 3 definiert sind, und $R^5$ Methyl oder Ethyl ist.

**9.** Das Verfahren nach Anspruch 7, worin das Amin 4-Amino-2,2,6,6-tetramethylpiperidin ist, das Monoester-Säurechlorid Ethyloxalylchlorid oder Ethylsuccinylchlorid ist, das Hydrazin 85%iges Hydrazinhydrat ist, das Lösungsmittel in Schritt (1) Methylenchlorid ist, und das Lösungsmittel in Schritt (2) Methanol ist.

**10.** Ein Verfahren zur Herstellung der Verbindung nach Anspruch 1 mit der Struktur

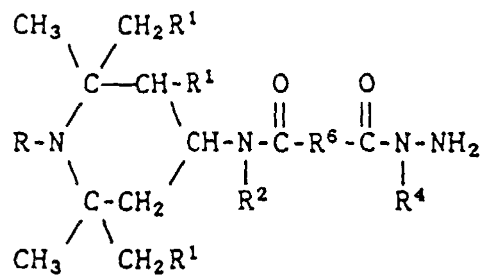

worin R, $R^1$, $R^2$ und $R^4$ wie zuvor in Anspruch 1 definiert sind, und $R^6$ eine direkte Bindung, ein zweibindiger Alkylen- oder verzweigter zweibindiger Alkylenrest mit einem Kohlenstoffatom oder 3 bis 14 Kohlenstoffatomen, ein zweibindiger Oxydialkylen- oder zweibindiger Thiodialkylenrest mit 4 bis 10 Kohlenstoffatomen oder ein zweibindiger o-, m- oder p-Phenylen- oder substituierter zweibindiger Phenylenrest ist, wobei die Substituenten $C_1$-$C_4$-Alkyl, $C_1$-$C_4$-Alkoxy, Hydroxy, Brom, Chlor, Mercapto oder $C_1$-$C_4$-Alkylmercapto sein können, durch (1) Umsetzung eines Amins der Formel

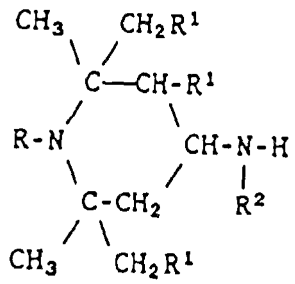

mit einem Dialkyl- oder Diphenyldiester der Formel

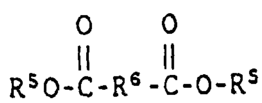

entweder unverdünnt oder in Gegenwart eines polaren Lösungsmittels, um ein Zwischenprodukt, das halb Ester halb Amid ist, der Formel

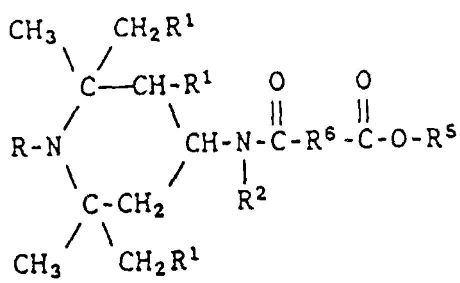

zu bilden, und anschließend (2) Umsetzung des Zwischenprodukts mit einem Hydrazin der Formel $R^4NH-NH_2$ und Isolierung eines Produkts der Formel

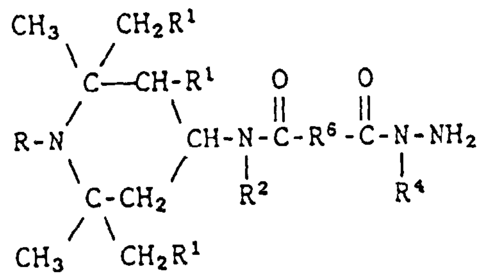

durch Kristallisation aus dem Lösungsmittel oder durch Eindampfen des Lösungsmittels, wobei R, $R^1$, $R^2$, $R^4$ und $R^6$ wie zuvor definiert sind, und $R^5$ wie in Anspruch 6 definiert ist.

**11.** Das Verfahren nach Anspruch 10, worin das Amin mit einem Dialkyl- oder Diphenyloxalat, worin $R^6$ eine direkte Bindung ist, unverdünnt oder in einem polaren Lösungsmittel umgesetzt wird, und worin dann das Zwischenprodukt, das halb Ester halb Amid ist, und worin $R^6$ eine direkte Bindung ist, mit einem Hydrazin in einem polaren Lösungsmittel umgesetzt und die Verbindung nach Anspruch 1, worin $R^3$ eine direkte Bindung ist, durch Kristallisation aus dem Lösungsmittel oder durch Eindampfen des Lösungsmittels isoliert wird.

**12.** Das Verfahren nach Anspruch 10, worin das Amin 4-Amino-2,2,6,6-tetramethylpiperidin ist, der Diester Dimethyladipat ist, das Hydrazin Hydrazinhydrat ist, und Schritt (1) unverdünnt durchgeführt wird, und die Hydrazinolyse in Methanol, Ethanol, Propanol oder Isopropanol durchgeführt wird.

**13.** Ein Verfahren nach Anspruch 11, worin das Amin 4-Amino-2,2,6,6-tetramethylpiperidin oder Bis(2, 2,6, 6-tetramethyl-4-piperidinyl)amin ist, der Oxalatdiester Dimethyl- oder Diethyloxalat ist, das Hydrazin 85%iges Hydrazinhydrat ist, und das Lösungsmittel Methanol, Ethanol, Propanol oder Isopropanol ist.

**14.** Ein Verfahren nach Anspruch 11, worin das Amin 4-Amino-2,2,6,6-tetramethylpiperidin ist, der Oxalatdiester Dimethyl- oder Diethyloxalat ist, das Lösungsmittel überschüssiger Oxalatdiester ist, der überschüssige Oxalatdiester entfernt und durch Methanol oder Ethanol ersetzt wird, bevor das Zwischenprodukt mit einem Hydrazin, das 85%iges Hydrazinhydrat ist, umgesetzt wird.

**15.** Die Verwendung einer Verbindung nach irgendeinem der Ansprüche 1 bis 5 zur Stabilisierung von Polymeren und Copolymeren.

**Patentansprüche für folgenden Vertragsstaat : ES**

**1.** Ein Verfahren zur Herstellung einer Verbindung der Formel

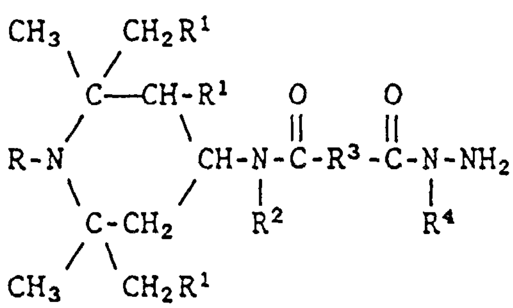

worin:

R ausgewählt ist aus Wasserstoff, Oxyl, Hydroxyl, Alkyl mit 1 bis 20 Kohlenstoffatomen, Alkenyl oder Alkinyl mit 3 bis 8 Kohlenstoffatomen, Aralkyl mit 7 bis 12 Kohlenstoffatomen, aliphatischem Acyl mit 1 bis 10 Kohlenstoffatomen, aromatischem Acyl mit 7 bis 13 Kohlenstoffatomen, Alkoxycarbonyl mit 2 bis 9 Kohlenstoffatomen, Aryloxycarbonyl mit 7 bis 15 Kohlenstoffatomen, alkyl-, aryl-, cycloalkyl- oder aralkylsubstituiertem Carbamoyl mit 2 bis 13 Kohlenstoffatomen, 2-Cyanoethyl, Hydroxyalkyl mit 1 bis 6 Kohlenstoffatomen, Epoxyalkyl mit 3 bis 10 Kohlenstoffatomen oder einem Polyalkylenoxid mit 4 bis 30 Kohlenstoffatomen,
$R^1$ ausgewählt ist aus Wasserstoff oder Niedrigalkyl mit 1 bis 4 Kohlenstoffatomen,
$R^2$ ausgewählt ist aus Wasserstoff, Alkyl mit 1 bis 10 Kohlenstoffatomen, Cycloalkyl mit 5 bis 12 Kohlenstoffatomen, Aralkyl mit 7 bis 12 Kohlenstoffatomen, Aryl mit 6 bis 12 Kohlenstoffatomen, 2-Cyanoethyl oder einem Rest der Formel

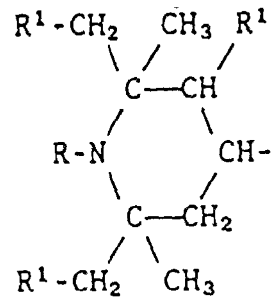

$R^3$ ausgewählt ist aus einer direkten Bindung, einem zweibindigen Alkylen- oder verzweigten zweibindigen Alkylenrest mit 1 bis 14 Kohlenstoffatomen, einem zweibindigen Alkenylenrest mit 2 bis 10 Kohlenstoffatomen, einem zweibindigen Oxydialkylen- oder zweibindigen Thiodialkylenrest mit 4 bis 10 Kohlenstoffatomen oder einem zweibindigen o-, m- oder p-Phenylen- oder substituierten zweibindigen Phenylenrest, wobei der Substituent $C_1$-$C_4$-Alkyl, $C_1$-$C_4$-Alkoxy, Hydroxy, Brom, Chlor, Mercapto oder $C_1$-$C_4$-Alkylmercapto sein kann, und
$R^4$ ausgewählt ist aus Wasserstoff, einer primären oder sekundären Alkylgruppe mit 1 bis 8 Kohlenstoffatomen, einer Aralkylgruppe mit 7 bis 12 Kohlenstoffatomen oder einer Cycloalkylgruppe mit 5 bis 12 Kohlenstoffatomen, durch (1) Umsetzung eines Amins der Formel

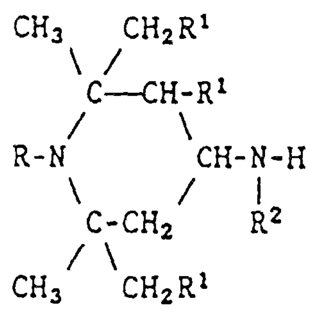

mit einem Monoester-Säurechlorid der Formel

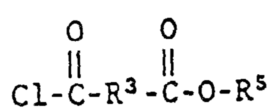

in einem inerten Lösungsmittel, wie z.B. einem Kohlenwasserstoff-, Chlorkohlenwasserstoff- oder Etherlösungsmittel, und anschließend (2) Umsetzung des Zwischenprodukts, das halb Ester halb Amid ist,

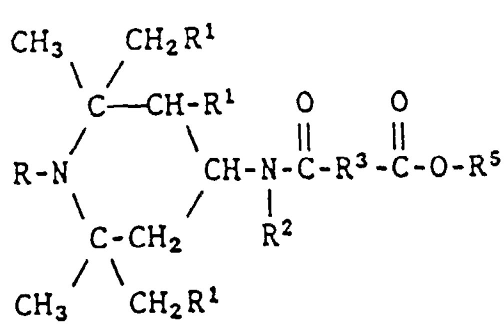

mit einem Hydrazin der Formel $R^4$-NH-$NH_2$ in einem polaren Lösungsmittel und Isolierung der Verbindung durch Kristallisation aus dem polaren Lösungsmittel oder durch Eindampfen des Lösungsmittels, wobei R, $R^1$,

$R^2$, $R^3$ und $R^4$ wie zuvor definiert sind, und $R^5$ Niedrigalkyl mit 1 bis 6 Kohlenstoffatomen oder Phenyl ist.

**2.** Das Verfahren nach Anspruch 1, worin R ausgewählt ist aus Wasserstoff, Alkyl mit 1 bis 4 Kohlenstoffatomen, Alkenyl mit 3 Kohlenstoffatomen, Benzyl, 2-Cyanoethyl oder Acetyl,

$R^1$ ausgewählt ist aus Wasserstoff oder Methyl,
$R^2$ ausgewählt ist aus Wasserstoff, Alkyl mit 1 bis 4 Kohlenstoffatomen oder 2,2,6,6-Tetramethyl-4-piperidinyl,
$R^3$ ausgewählt ist aus einer direkten Bindung, einem zweibindigen Alkylenrest mit 1 bis 8 Kohlenstoffatomen oder einem zweibindigen o-, m- oder p-Phenylenrest, und
$R^4$ ausgewählt ist aus Wasserstoff, primärem oder sekundärem Alkyl mit 1 bis 4 Kohlenstoffatomen, Benzyl oder Cyclohexyl, und
$R^5$ Methyl oder Ethyl ist, das Lösungsmittel in Schritt (1) ein Kohlenwasserstoff- oder Chlorkohlenwasserstofflösungsmittel ist, und das Lösungsmittel in Schritt (2) ein Alkohol mit niedrigem Molekulargewicht oder Glycol ist.

**3.** Das Verfahren nach Anspruch 2, worin

R ausgewählt ist aus Wasserstoff, Methyl oder Acetyl,
$R^1$ Wasserstoff ist,
$R^2$ ausgewählt ist aus Wasserstoff oder 2,2,6,6-Tetramethyl-4-piperidinyl,
$R^3$ ausgewählt ist aus einer direkten Bindung oder einem zweibindigen 1,2-Ethylenrest, und
$R^4$ Wasserstoff oder Methyl ist, und
$R^5$ Methyl oder Ethyl ist.

**4.** Das Verfahren nach Anspruch 3, worin R, $R^1$, $R^2$ und $R^4$ Wasserstoff sind, $R^3$ eine direkte Bindung oder ein zweibindiger Alkylenrest mit 1 bis 8 Kohlenstoffatomen ist, insbesondere ein zweibindiger 1,2-Ethylenrest, $R^5$ Ethyl ist, und das Hydrazin 85%iges Hydrazinhydrat ist, das Lösungsmittel in Schritt (1) Methylenchlorid ist, und das Lösungsmittel in Schritt (2) Methanol ist.

**5.** Das Verfahren nach Anspruch 3, worin $R^1$, $R^2$ und $R^4$ Wasserstoff sind, R Methyl oder Acetyl ist, und $R^3$ eine direkte Bindung ist.

**6.** Ein Verfahren nach Anspruch 1 zur Herstellung einer Verbindung mit der Struktur

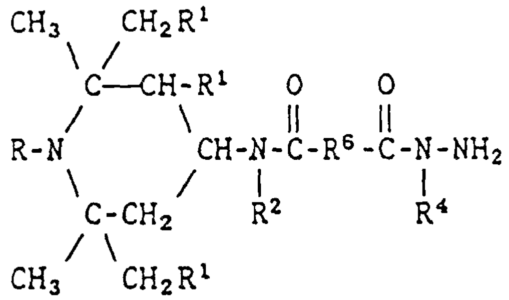

worin R, $R^1$, $R^2$ und $R^4$ wie zuvor in Anspruch 1 definiert sind, und $R^6$ eine direkte Bindung, ein zweibindiger Alkylen- oder verzweigter zweibindiger Alkylenrest mit einem Kohlenstoffatom oder 3 bis 14 Kohlenstoffatomen, ein zweibindiger Oxydialkylen- oder zweibindiger Thiodialkylenrest mit 4 bis 10 Kohlenstoffatomen oder ein zweibindiger o-, m- oder p-Phenylen- oder substituierter zweibindiger Phenylenrest ist, wobei die Substituenten $C_1$-$C_4$-Alkyl, $C_1$-$C_4$-Alkoxy, Hydroxy, Brom, Chlor, Mercapto oder $C_1$-$C_4$-Alkylmercapto sein können, durch (1) Umsetzung eines Amins der Formel

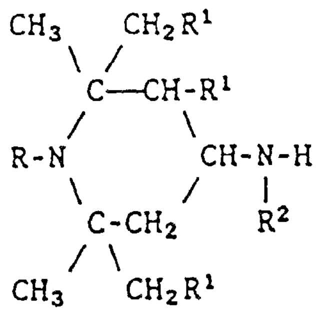

mit einem Dialkyl- oder Diphenyldiester der Formel

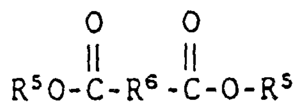

entweder unverdünnt oder in Gegenwart eines polaren Lösungsmittels, um ein Zwischenprodukt, das halb Ester halb Amid ist, der Formel

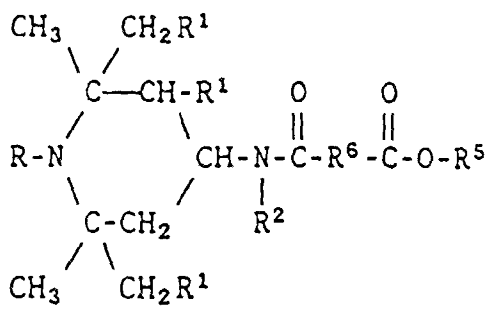

zu bilden, und anschließend (2) Umsetzung des Zwischenprodukts mit einem Hydrazin der Formel $R^4NH\text{-}NH_2$ und Isolierung eines Produkts der Formel

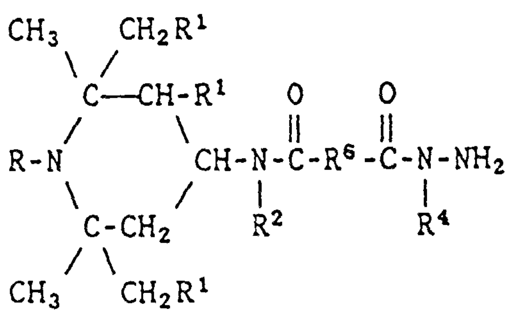

durch Kristallisation aus dem Lösungsmittel oder durch Eindampfen des Lösungsmittels, wobei R, $R^1$, $R^2$, $R^4$ und $R^6$ wie zuvor definiert sind, und $R^5$ wie in Anspruch 1 definiert ist.

**7.** Das Verfahren nach Anspruch 6, worin das Amin mit einem Dialkyl- oder Diphenyloxalat, worin $R^6$ eine direkte Bindung ist, unverdünnt oder in einem polaren Lösungsmittel umgesetzt wird, und worin dann das Zwischenprodukt, das halb Ester halb Amid ist, und worin $R^6$ eine direkte Bindung ist, mit einem Hydrazin in einem polaren Lösungsmittel umgesetzt und die Verbindung nach Anspruch 1, worin $R^3$ eine direkte Bindung ist, durch Kristallisation aus dem Lösungsmittel oder durch Eindampfen des Lösungsmittels isoliert wird.

**8.** Das Verfahren nach Anspruch 6, worin das Amin 4-Amino-2,2,6,6-tetramethylpiperidin ist, der Diester Dimethyladipat ist, das Hydrazin Hydrazinhydrat ist, und Schritt (1) unverdünnt durchgeführt wird, und die Hydrazinolyse in Methanol, Ethanol, Propanol oder Isopropanol durchgeführt wird.

**9.** Ein Verfahren nach Anspruch 7, worin das Amin 4-Amino-2,2,6, 6-tetramethylpiperidin oder Bis(2,2,6,6-tetramethyl-4-piperidinyl)amin ist, der Oxalatdiester Dimethyl- oder Diethyloxalat ist, das Hydrazin 85%iges Hydrazinhydrat ist, und das Lösungsmittel Methanol, Ethanol, Propanol oder Isopropanol ist.

**10.** Ein Verfahren nach Anspruch 7, worin das Amin 4-Amino-2,2,6,6-tetramethylpiperidin ist, der Oxalatdiester Dimethyl- oder Diethyloxalat ist, das Lösungsmittel überschüssiger Oxalatdiester ist, der überschüssige Oxalatdiester entfernt und durch Methanol oder Ethanol ersetzt wird, bevor das Zwischenprodukt mit einem Hydrazin, das 85%iges Hydrazinhydrat ist, umgesetzt wird.

**11.** Die Verwendung einer Verbindung, die gemäß den Verfahren nach irgendeinem der Ansprüche 1 bis 10 hergestellt ist, zur Stabilisierung von Polymeren und Copolymeren.

**Revendications**

**Revendications pour les Etats contractants suivants : BE, CH, DE, FR, GB, IT, LI, NL, SE**

**1.** Composé de formule

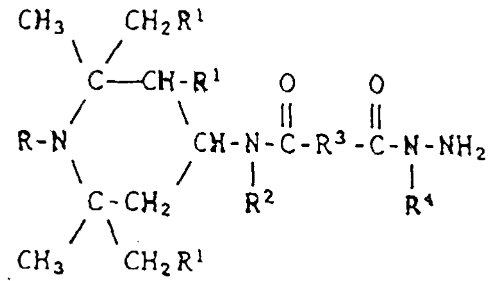

dans laquelle :

R est choisi entre l'hydrogène, des groupes oxyle, hydroxyle, alkyle ayant 1 à 20 atomes de carbone, alcényle ou alcynyle ayant 3 à 8 atomes de carbone, aralkyle ayant 7 à 12 atomes de carbone, acyle aliphatique ayant 1 à 10 atomes de carbone, acyle aromatique ayant 7 à 13 atomes de carbone, alkoxycarbonyle ayant 2 à 9 atomes de carbone, aryloxycarbonyle ayant 7 à 15 atomes de carbone, carbamoyle de 2 à 13 atomes de carbone à substituant alkyle, aryle, cycloalkyle ou aralkyle, 2-cynaoéthyle, hydroxyalkyle ayant 1 à 6 atomes de carbone, époxyalkyle ayant 3 à 10 atomes de carbone ou un polymère d'oxyde d'alkylène de 4 à 30 atomes de carbone,

$R^1$ est choisi entre l'hydrogène et un groupe alkyle inférieur ayant 1 à 4 atomes de carbone,

$R^2$ est choisi entre l'hydrogène, des groupes alkyle ayant 1 à 10 atomes de carbone, cycloalkyle ayant 5 à 12 atomes de carbone, aralkyle ayant 7 à 12 atomes de carbone, aryle ayant 6 à 12 atomes de carbone, 2-cyanoéthyle et un radical de formule

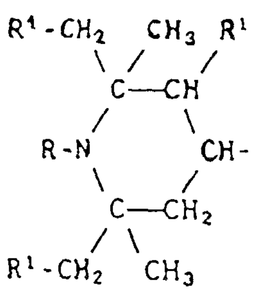

$R^3$ est choisi entre une liaison directe, un diradical alkylène ou alkylène ramifié ayant 1 à 14 atomes de carbone, un diradical alcénylène ayant 2 à 10 atomes de carbone, un diradical oxydialkylène ou thiodialkylène ayant 4 à 10 atomes de carbone ou un diradical o-, m- ou p-phénylène ou phénylène substitué dont le substituant peut être un groupe alkyle en $C_1$ à $C_4$, alkoxy en $C_1$ à $C_4$, hydroxy, bromo, chloro, mercapto ou alkylmercapto en $C_1$ à $C_4$, et

$R^4$ est choisi entre l'hydrogène, un groupe alkyle primaire ou secondaire ayant 1 à 8 atomes de carbone, un

groupe aralkyle ayant 7 à 12 atomes de carbone, et un groupe cycloalkyle ayant 5 à 12 atomes de carbone.

**2.** Composé suivant la revendication 1, dans lequel R est choisi entre l'hydrogène, un groupe alkyle ayant 1 à 4 atomes de carbone, un groupe alcényle ayant 3 atomes de carbone, un groupe benzyle, un groupe 2-cyanoéthyle et un groupe acétyle,

$R^1$ est choisi entre l'hydrogène et un groupe méthyle,
$R^2$ est choisi entre l'hydrogène, un groupe alkyle ayant 1 à 4 atomes de carbone et le groupe 2,2,6,6-tétraméthyl-4-pipéridinyle,
$R^3$ est choisi entre une liaison directe, un diradical alkylène ayant 1 à 8 atomes de carbone et un diradical o-, m- ou p-phénylène, et
$R^4$ est choisi entre l'hydrogène, un groupe alkyle primaire ou secondaire ayant 1 à 4 atomes de carbone, un groupe benzyle et un groupe cyclohexyle.

**3.** Composé suivant la revendication 2, dans lequel

R est choisi entre l'hydrogène, un groupe méthyle et un groupe acétyle,
$R^1$ représente l'hydrogène,
$R^2$ est choisi entre l'hydrogène et le groupe 2,2,6,6-tétraméthyl-4-pipéridinyle,
$R^3$ est choisi entre une liaison directe et un diradical 1,2-éthylène, et
$R^4$ représente l'hydrogène ou un groupe méthyle.

**4.** Composé suivant la revendication 3, dans lequel

R, $R^1$, $R^2$ et $R^4$ représentent l'hydrogène et $R^3$ représente une liaison directe ou un diradical alkylène ayant 1 à 8 atomes de carbone, notamment un diradical 1,2-éthylène.

**5.** Composé suivant la revendication 3, dans lequel $R^1$, $R^2$ et $R^4$ représentent l'hydrogène, R représente un groupe méthyle ou acétyle et $R^3$ représente une liaison directe.

**6.** Procédé de préparation d'un composé suivant la revendication 1, par (1) réaction d'une amine de formule

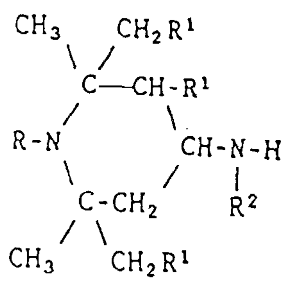

avec un chlorure d'acide monoester de formule

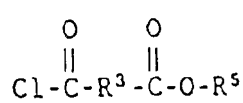

dans un solvant inerte tel qu'un hydrocarbure, un hydrocarbure chloré ou un éther, et ensuite par (2) réaction de l'hémiester-semi-amide intermédiaire

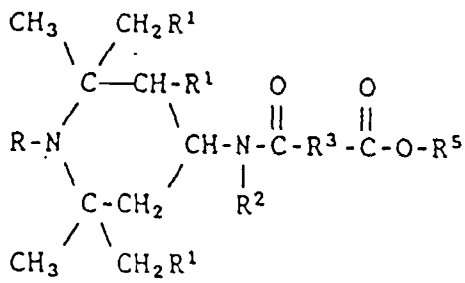

avec une hydrazine de formule $R^4$-NH-$NH_2$ dans un solvant polaire et isolement du composé suivant la revendication 1 par cristallisation de solvant polaire ou par évaporation du solvant, formules dans lesquelles R, $R^1$, $R^2$, $R^3$ et $R^4$ répondent aux définitions mentionnées précédemment dans la revendication 1 et $R^5$ représente un groupe alkyle inférieur ayant 1 à 6 atomes de carbone ou un groupe phényle.

**7.** Procédé suivant la revendication 6, dans lequel R, $R^1$, $R^2$, $R^3$ et $R^4$ répondent aux définitions mentionnées précédemment dans la revendication 2, $R^5$ représente un groupe méthyle ou éthyle, le solvant à l'étape (1) est un hydrocarbure ou hydrocarbure chloré et le solvant dans l'étape (2) est un alcool de bas poids moléculaire ou glycol.

**8.** Procédé suivant la revendication 7, dans lequel $R^1$, $R^2$, $R^3$ et $R^4$ répondent aux définitions mentionnées précédemment dans la revendication 3 et $R^5$ représente un groupe méthyle ou éthyle.

**9.** Procédé suivant la revendication 7, dans lequel l'amine consiste en 4-amino-2,2,6,6-tétraméthyl-pipéridine, le chlorure d'acide monoester consiste en chlorure d'oxyalyléthyle ou chlorure de succinyléthyle, l'hydrazine consiste en hydrate d'hydrazine à 85 %, le solvant dans l'étape (1) consiste en chlorure de méthylène et le solvant dans l'étape (2) consiste en méthanol.

**10.** Procédé de préparation d'un composé suivant la revendication 1, ayant la structure

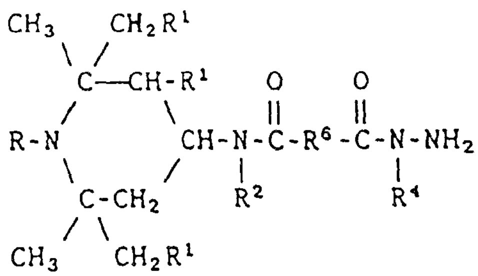

dans laquelle R, $R^1$, $R^2$, $R^3$ et $R^4$ répondent aux définitions mentionnées précédemment dans la revendication 1 et $R^6$ représente une liaison directe, un diradical alkylène ou alkylène ramifié ayant 1 atome de carbone ou bien 3 à 14 atomes de carbone, un diradical oxydialkylène ou thiodialkylène ayant 4 à 10 atomes de carbone ou un diradical o-, m- ou p-phénylène ou phénylène substitué dont les substituants peuvent être des groupes alkyle en $C_1$ à $C_4$, alkoxy en $C_1$ à $C_4$, hydroxy, bromo, chloro, mercapto ou alkylmercapto en $C_1$ à $C_4$, par (1) réaction d'une amine de formule

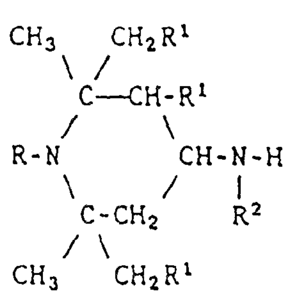

avec un diester de dialkyle ou de diphényle de formule

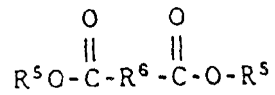

à l'état pur ou en présence d'un solvant polaire pour former un hémiester-semi-amide intermédiaire de formule

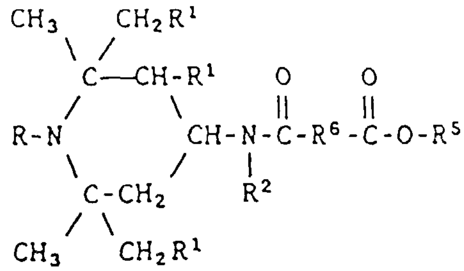

et ensuite par (2) réaction de l'intermédiaire avec une hydrazine de formule $R^4NH\text{-}NH_2$ et isolement d'un produit de formule

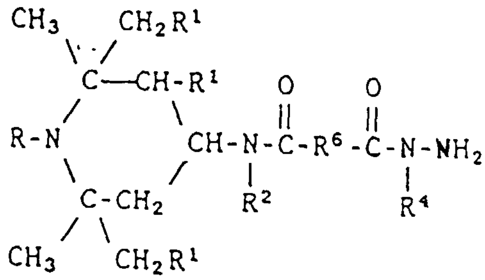

par cristallisation dans le solvant ou évaporation du solvant, formules dans lesquelles, R, $R^1$, $R^2$, $R^4$ et $R^6$ répondent aux définitions précitées et $R^5$ répond à la définition suivant la revendication 6.

**11.** Procédé suivant la revendication 10, dans lequel l'amine est amenée à réagir avec un oxalate de dialkyle ou de diphényle dans lequel $R^6$ représente une liaison directe, à l'état pur ou dans un solvant polaire, puis l'hémiester-semi-amide intermédiaire dans lequel $R^6$ représente une liaison directe est amené à réagir avec une hydrazine dans un solvant polaire, et le composé suivant la revendication 1, dans lequel $R^3$ représente une liaison directe est isolé par cristallisation dans le solvant ou par évaporation du solvant.

**12.** Procédé suivant la revendication 10, dans lequel l'amine consiste en 4-amino-2,2,6,6-tétraméthyl-pipéridine, le diester consiste en adipate de diméthyle, l'hydrazine consiste en hydrate d'hydrazine et l'étape (1) est mise en oeuvre à l'état pur et l'hydrazinolyse est effectuée dans le méthanol, l'éthanol, le propanol ou l'isopropanol.

**13.** Procédé suivant la revendication 11, dans lequel l'amine consiste en 4-amino-2,2,6,6-tétraméthyl-pipéridine ou bis-(2,2,6,6-tétraméthyl-4-pipéridinyl)amine, le diester consistant en oxalate est l'oxalate de diméthyle ou diéthyle, l'hydrazine consiste en hydrate d'hydrazine à 85% et le solvant consiste en méthanol, éthanol, propanol ou isopropanol.

**14.** Procédé suivant la revendication 11, dans lequel l'amine consiste en 4-amino-2,2,6,6-tétraméthyl-pipéridine, le diester consistant en un oxalate est l'oxalate de diméthyle ou diéthyle, le solvant consiste en le diester du type oxalate en excès, le diester du type oxalate en excès est éliminé et est remplacé par le méthanol ou l'éthanol avant la réaction d'un intermédiaire avec une hydrazine qui consiste en hydrate d'hydrazine à 85%.

**15.** Utilisation d'un composé suivant l'une quelconque des revendications 1 à 5 pour la stabilisation de polymères et copolymères.

**Revendications pour l'Etat contractant suivant : ES**

**1.** Procédé de préparation d'un composé de formule

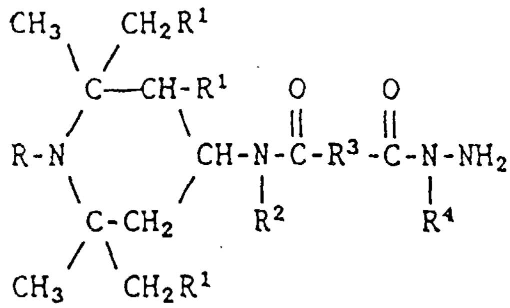

dans laquelle :

R est choisi entre l'hydrogène, des groupes oxyle, hydroxyle, alkyle ayant 1 à 20 atomes de carbone, alcényle ou alcynyle ayant 3 à 8 atomes de carbone, aralkyle ayant 7 à 12 atomes de carbone, acyle aliphatique ayant 1 à 10 atomes de carbone, acyle aromatique ayant 7 à 13 atomes de carbone, alkoxycarbonyle ayant 2 à 9 atomes de carbone, aryloxycarbonyle ayant 7 à 15 atomes de carbone, carbamoyle de 2 à 13 atomes de carbone à substituant alkyle, aryle, cycloalkyle, aralkyle, 2-cynaoéthyle, hydroxyalkyle ayant 1 à 6 atomes de carbone, époxyalkyle ayant 3 à 10 atomes de carbone et un polymère d'oxyde d'alkylène de 4 à 30 atomes de carbone,

$R^1$ est choisi entre l'hydrogène et un groupe alkyle inférieur ayant 1 à 4 atomes de carbone,

$R^2$ est choisi entre l'hydrogène, des groupes alkyle ayant 1 à 10 atomes de carbone, cycloalkyle ayant 5 à 12 atomes de carbone, aralkyle ayant 7 à 12 atomes de carbone, aryle ayant 6 à 12 atomes de carbone, 2-cyanoéthyle et un radical de formule

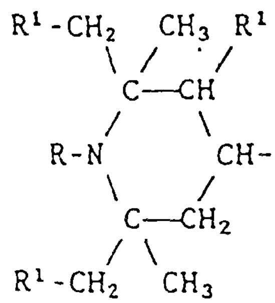

$R^3$ est choisi entre une liaison directe, un diradical alkylène ou alkylène ramifié ayant 1 à 14 atomes de carbone, un diradical alkylène ayant 2 à 10 atomes de carbone, un diradical oxydialkylène ou thiodialkylène ayant 4 à 10 atomes de carbone ou un diradical o-, m- ou p-phénylène ou phénylène substitué dont le substituant peut être un groupe alkyle en $C_1$ à $C_4$, alkoxy en $C_1$ à $C_4$, hydroxy, bromo, chloro, mercapto ou alkylmercapto en $C_1$ à $C_4$, et

$R^4$ est choisi entre l'hydrogène, un groupe alkyle primaire ou secondaire ayant 1 à 8 atomes de carbone, un groupe aralkyle ayant 7 à 12 atomes de carbone, et un groupe cycloalkyle ayant 5 à 12 atomes de carbone par (1) réaction d'une amine de formule

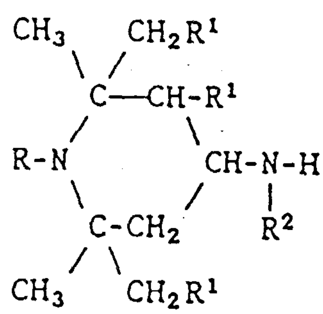

avec un chlorure d'acide-monoester de formule

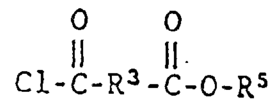

dans un solvant inerte tel qu'un hydrocarbure, un hydrocarbure chloré ou un éther, et ensuite par (2) réaction de l'hémiester-semi-amide intermédiaire

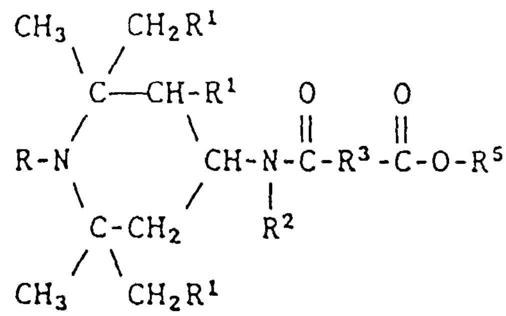

avec une hydrazine de formule $R^4NH\text{-}NH_2$ dans un solvant polaire et isolement du composé par cristallisation dans le solvant polaire ou par évaporation du solvant, formules dans lesquelles R, $R^1$, $R^2$, $R^3$ et $R^4$ répondent aux définitions précitées et $R^5$ représente un groupe alkyle inférieur ayant 1 à 6 atomes de carbone ou un groupe phényle.

**2.** Procédé suivant la revendication 1, dans lequel R est choisi entre l'hydrogène, des groupes alkyle ayant 1 à 4 atomes de carbone, alcényle ayant 3 atomes de carbone, benzyle, 2-cyanoéthyle et acétyle,

$R^1$ est choisi entre l'hydrogène et un groupe méthyle,
$R^2$ est choisi entre l'hydrogène, un groupe alkyle ayant 1 à 4 atomes de carbone et le groupe 2,2,6,6-tétraméthyl-4-pipéridinyle,
$R^3$ est choisi entre une liaison directe, un diradical alkylène ayant 1 à 8 atomes de carbone et un diradical o-, m- ou p-phénylène, et
$R^4$ est choisi entre l'hydrogène, un groupe alkyle primaire ou secondaire ayant 1 à 4 atomes de carbone, un groupe benzyle et un groupe cyclohexyle, et
$R^5$ représente un groupe méthyle ou éthyle, le solvant dans l'étape (1) est un hydrocarbure ou hydrocarbure chloré et le solvant dans l'étape (2) est un alcool de bas poids moléculaire ou glycol.

**3.** Procédé suivant la revendication 2, dans lequel

R est choisi entre l'hydrogène, un groupe méthyle et un groupe acétyle,
$R^1$ représente l'hydrogène,
$R^2$ est choisi entre l'hydrogène et le groupe 2,2,6,6-tétraméthyl-4-pipéridinyle,
$R^3$ est choisi entre une liaison directe, un diradical 1,2-éthylène,
$R^4$ est choisi entre l'hydrogène ou un groupe méthyle, et
$R^5$ représente un groupe méthyle ou éthyle.

**4.** Procédé suivant la revendication 3, dans lequel

R, $R^1$, $R^2$ et $R^4$ représentent l'hydrogène et $R^3$ représente une liaison directe ou un diradical alkylène ayant 1 à 8 atomes de carbone, notamment un diradical 1,2-éthylène, $R^5$ représente un groupe éthyle et l'hydrazine consiste en hydrate d'hydrazine à 85%, le solvant dans l'étape (1) consiste en chlorure de méthylène et le solvant de l'étape (2) consiste en méthanol.

**5.** Procédé suivant la revendication 3, dans lequel $R^1$, $R^2$ et $R^4$ représentent l'hydrogène, R représente un groupe méthyle ou actyle et $R^3$ représente une liaison directe.

**6.** Procédé suivant la revendication 1 pour la préparation d'un composé de structure

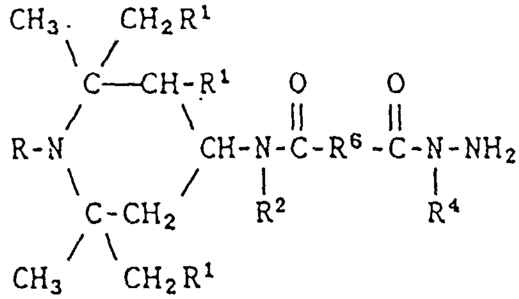

dans laquelle R, $R^1$, $R^2$ et $R^4$ répondent aux définitions mentionnées précédemment dans la revendication 1 et $R^6$ représente une liaison directe, un diradical alkylène ou alkylène ramifié ayant 1 atome de carbone ou bien 3 à 14 atomes de carbone, un diradical oxydialkylène ou thiodialkylène ayant 4 à 10 atomes de carbone ou un diradical o-, m- ou p-phénylène ou phénylène substitué dans lequel les substituants peuvent être des groupes alkyle en $C_1$ à $C_4$, alkoxy en $C_1$ à $C_4$, hydroxy, bromo, chloro, mercapto ou alkylmercapto en $C_1$ à $C_4$, par (1) réaction d'une amine de formule

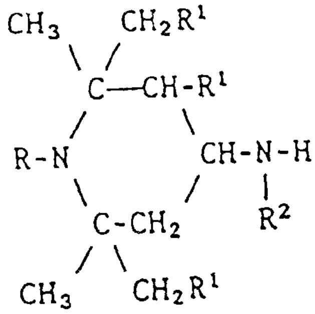

avec un diester de dialkyle ou de diphényle de formule

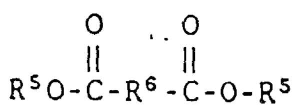

à l'état pur ou en présence d'un solvant polaire pour former un hémiester-semi-amide intermédiaire de formule

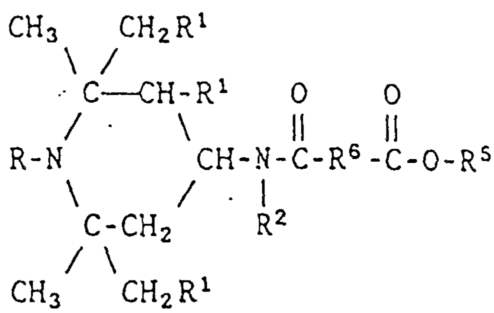

et ensuite par (2) réaction de l'intermédiaire avec une hydrazine de formule $R^4NH-NH_2$ et isolement d'un produit de formule

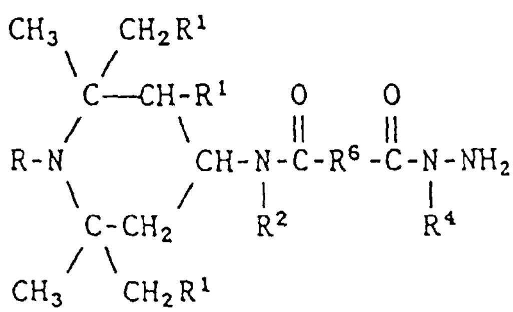

par cristallisation dans le solvant ou par évaporation du solvant, formules dans lesquelles R, $R^1$, $R^2$, $R^4$ et $R^6$ répondent aux définitions précitées et $R^5$ répond à la définition suivant la revendication 1.

**7.** Procédé suivant la revendication 6, dans lequel l'amine est amenée à réagir avec un oxalate de dialkyle ou de diphényle dans lequel $R^6$ représente une liaison directe, à l'état pur ou dans un solvant polaire, et l'hémiester-semi-amide dans lequel $R^6$ représente une liaison directe est ensuite amené à réagir avec une hydrazine dans un solvant polaire, et le composé suivant la revendication 1, dans lequel $R^3$ représente une liaison directe est isolé par crisallisation dans le solvant ou par évaporation du solvant.

**8.** Procédé suivant la revendication 6, dans lequel l'amine consiste en 4-amino-2,2,6,6-tétraméthyl-pipéridine, le diester consistant en adipate de diméthyle, l'hydrazine consistant en hydrate d'hydrazine et l'étape (1) est mise en oeuvre à l'état pur et l'hydrazinolyse est effectuée dans le méthanol, l'éthanol, le propanol ou l'isopropanol.

**9.** Procédé suivant la revendication 7, dans lequel l'amine consiste en 4-amino-2,2,6,6-tétraméthyl-pipéridine ou bis-2,2,6,6-tétraméthyl-4-pipéridinylamine, le diester consistant en un oxalate est l'oxalate de diméthyle ou de diéthyle, l'hydrazine consiste en hydrate d'hydrazine à 85% et le solvant consistant en méthanol, éthanol, propanol ou isopropanol.

**10.** Procédé suivant la revendication 7, dans lequel l'amine consiste en 4-amino-2,2,6,6-tétraméthyl-pipéridine, le diester consistant en un oxalate est l'oxalate de diméthyle ou de diéthyle, le solvant consiste en diester du type oxalate en excès, le diester du type oxalate en excès est éliminé et est remplacé par le méthanol ou l'éthanol avant réaction d'un intermédiaire avec une hydrazine qui consiste en hydrate d'hydrazine à 85%.

**11.** Utilisation d'un composé préparé par les procédés suivant l'une quelconque des revendications 1 à 10 pour la stabilisation de polymères et copolymères.